# EUROPEAN PATENT SPECIFICATION

(11) **EP 2 307 554 B1**
(45) Date of publication and mention of the grant of the patent: **26.06.2013**
(21) Application number: 09802477.1
(22) Date of filing: 10.07.2009
(51) Int. Cl.: C12P 7/10, C12P 19/02

(54) **Process for oxidizing lignocellulosic material employing a Marasmius scorodonius peroxidase**
Verfahren zur Oxidation von lignocellulosehaltigem Material unter Verwendung einer Marasmius scorodonius Peroxidase
Procédé d'oxydation d'une matière lignocellulosique en utilisant une péroxidase de Marasmius scorodonius

(30) Priority: 29.07.2008 EP 08161376
(43) Date of publication of application: 13.04.2011
(73) Proprietor: DSM IP Assets B.V., 6411 TE Heerlen (NL)
(72) Inventor: ZORN, Holger, 35435 Wettenberg (DE); SZWEDA, Renate, 58636 Iserlohn (DE); KUMAR, Manoj, Fremont California 94555 (US); WILMS, Johannes, NL-1511 WG Oostzaan (NL)
(74) Representative: Kleiborn, Paul Erik
(86) International application number: PCT/EP2009/058871
(87) International publication number: WO 2010/012579

(56) References cited:
- US-A- 5 865 898
- US-A1- 2007 077 630
- L/OBARZEWSKI J ET AL: "Lignocellulose biotransformation with immobilized cellulase, d-glucose oxidase and fungal peroxidases" ENZYME AND MICROBIAL TECHNOLOGY, STONEHAM, MA, US, vol. 7, no. 11, 1 November 1985 (1985-11-01), pages 564-566, XP023678843 ISSN: 0141-0229 [retrieved on 1985-11-01]
- STEFFEN KARI T ET AL: "Differential degradation of oak (Quercus petraea) leaf litter by litter-decomposing basidiomycetes" RESEARCH IN MICROBIOLOGY, vol. 158, no. 5, June 2007 (2007-06), pages 447-455, XP002509247 ISSN: 0923-2508
- SCHEIBNER MANUELA ET AL: "Novel peroxidases of Marasmius scorodonius degrade beta-carotene" APPLIED MICROBIOLOGY AND BIOTECHNOLOGY, vol. 77, no. 6, January 2008 (2008-01), pages 1241-1250, XP002509248 ISSN: 0175-7598
- JOHJIMA T ET AL: "Isolation and cDNA cloning of novel hydrogen peroxide-dependent phenol oxidase from the basidiomycete Termitomyces albuminosus" APPLIED MICROBIOLOGY AND BIOTECHNOLOGY, SPRINGER VERLAG, BERLIN, DE, vol. 61, no. 3, 27 February 2003 (2003-02-27), pages 220-225, XP002387966 ISSN: 0175-7598
- HAMMEL KENNETH E ET AL: "Role of fungal peroxidases in biological ligninolysis" CURRENT OPINION IN PLANT BIOLOGY, vol. 11, no. 3, June 2008 (2008-06), pages 349-355, XP002509249 ISSN: 1369-5266

## Description

### Field of the invention

The present invention relates to methods for the oxidation of a lignocellulosic material comprising a non-starch carbohydrate material, for increasing the susceptibility of such material to enzymatic degradation, for the degradation of such material and for producing a sugar or sugars from such material using peroxidase enzymes. The invention also relates to the use of peroxidase enzymes in such methods.

### Background to the invention

Carbohydrates constitute the most abundant organic compounds on earth. However, much of this carbohydrate is sequestered in complex polymers including starch (the principle storage carbohydrate in seeds and grain), and a. collection of carbohydrates and lignin known as lignocellulose. The main carbohydrate components of lignocellulose are cellulose, hemicellulose, and pectins. These complex polymers are often referred to collectively as lignocellulose.

Bioconversion of renewable lignocellulosic biomass to a fermentable sugar that is subsequently fermented to produce alcohol (e.g., ethanol) as an alternative to liquid fuels has attracted an intensive attention of researchers since 1970s, when the oil crisis broke out because of decreasing the output of petroleum by OPEC. Ethanol has been widely used as a 10% blend to gasoline in the USA or as a neat fuel for vehicles in Brazil in the last two decades. More recently, the use of E85, an 85% ethanol blend has been implemented especially for clean city applications. The importance of fuel bioethanol will increase in parallel with increases in prices for oil and the gradual depletion of its sources. Additionally, fermentable sugars are being used to produce plastics, polymers and other biobased products and this industry is expected to grow substantially therefore increasing the demand for abundant low cost fermentable sugars which can be used as a feed stock in lieu of petroleum based feedstocks.

The sequestration of such large amounts of carbohydrates in plant biomass provides a plentiful source of potential energy in the form of sugars, both five carbon and six carbon sugars that could be utilized for numerous industrial and agricultural processes. However, the enormous energy potential of these carbohydrates is currently under-utilized because the sugars are locked in complex polymers, and hence are not readily accessible for fermentation. Methods that generate sugars from plant biomass would provide plentiful, economically-competitive feedstocks for fermentation into chemicals, plastics, and fuels.

In spite of the continued research of the last few decades to understand enzymatic lignocellulosic biomass degradation and cellulase production, it remains desirable to discover or to engineer new highly active cellulases and hemicellulases. It would also be highly desirable to construct highly efficient enzyme compositions capable of performing rapid and efficient biodegradation of lignocellulosic materials.

In addition, chemo-mechanical processes are typically used so as to free cellulose for subsequent conversion to fermentable sugars. Such chemo-mechanical processes require expensive reaction vessels and are energy intensive. Moreover, chemical pre-treatment occurring at high temperatures and extreme pH conditions are not compatible with known cellulose-degrading enzymes. Further, these reactions produce compounds that must be removed before fermentation can proceed. As a result, chemical pretreatment processes currently occur in separate reaction vessels from cellulose degradation and must occur prior to cellulose degradation.

The document US 5,865,898 A discloses a method for oxidizing a lignocellulosic material, which method comprises contacting said lignocellulosic material with an oxidizing agent selected from oxygen and oxygen containing gases.

Thus, methods that are more compatible with the cellulose degradation process, do not required high temperature and pressure, do not generate toxic waste products and require less energy are desirable.

### Summary of the invention

We have shown that peroxidases of *Marasmius scorodonius* can help cellulases to release greater amounts of sugar from lignocellulosic feedstock than would be the case in the absence of said peroxidases.

According to the invention, there is thus provided a method for the oxidation of a lignocellulosic material comprising a non-starch carbohydrate, which method comprises contacting said lignocellulosic material comprising a non-starch carbohydrate with a polypeptide which has peroxidase activity and which is:
a. a polypeptide comprising an amino acid sequence having at least 55% monology with an amino acid sequence set out in any one of: amino acids 21 to 513 of SEQ NO: 2; amino acids 20 to 510 of SEQ ID NO: 4; amino acids 1 to 493 of SEQ ID NO 6; or amino acids 1 to 491 of SEQ ID NO: 8 or
b. a polypeptide encoded by a polynucleotide comprising a nucleotide sequence having at least 55% homology with the nucleotide sequence set out in any one of: nucleotides 61 to 1539 of SEQ ID NO: 1; nucleotides 58 to 1530 of SEQ ID NO: 3; nucleotides 1 to 1479 of SEQ ID NO: 5; or nucleotides 1 to 1473 of SEQ ID NO: 7,
thereby to oxidize said lignocellulosic material comprising a non-starch carbohydrate.

The invention also provides:
- a method for increasing the susceptibility of a lignocellulosic material comprising a non-starch carbohydrate to enzymatic degradation, which method comprises contacting said lignocellulosic material comprising a non-starch carbohydrate with a polypeptide which has peroxidase activity and which is:
   a. a polypeptide comprising an amino acid sequence having at least 55% homology with an amino acid sequence set out in any one of: amino acids 21 to 513 of SEQ NO: 2; amino acids 20 to 510 of SEQ ID NO: 4; amino acids 1 to 493 of SEQ ID NO 6; or amino acids 1 to 491 of SEQ ID NO: 8 or
   b. a polypeptide encoded by a polynucleotide comprising a nucleotide sequence having at least 55% homology with the nucleotide sequence set out in any one of: nucleotides 61 to 1539 of SEQ ID NO: 1; nucleotides 58 to 1530 of SEQ ID NO: 3; nucleotides 1 to 1479 of SEQ ID NO: 5; or nucleotides 1 to 1473 of SEQ ID NO: 7,
      thereby to increase the susceptibility of a lignocellulosic material comprising a non-starch carbohydrate to enzymatic degradation;
- a method for producing a sugar or sugars from a lignocellulosic material comprising a non-starch carbohydrate, which method comprises:
   (i) oxidizing said lignocellulosic material comprising a non-starch carbohydrate or increasing the susceptibility of said lignocellulosic material comprising a non-starch carbohydrate to enzymatic degradation using a method as described above; and
   (ii) contacting the thus-modified lignocellulosic material comprising a non-starch carbohydrate with a cellulase and/or a hemicellulase and/or a pectinase,
      thereby to produce a sugar or sugars from a lignocellulosic material comprising a non-starch carbohydrate; and
- a method for producing a sugar or sugars from a lignocellulosic material which comprises a non-starch carbohydrate, which method comprises contacting said lignocellulosic material comprising a non-starch carbohydrate with:
   (i) a polypeptide which has peroxidase activity and which is:
      a. a polypeptide comprising an amino acid sequence having at least 55% homology with an amino acid sequence set out in any one of: amino acids 21 to 513 of SEQ NO: 2; amino acids 20 to 510 of SEQ ID NO: 4; amino acids 1 to 493 of SEQ ID NO 6; or amino acids 1 to 491 of SEQ ID NO: 8 or
      b. a polypeptide encoded by a polynucleotide comprising a nucleotide sequence having at least 55% homology with the nucleotide sequence set out in any one of: nucleotides 61 to 1539 of SEQ ID NO: 1; nucleotides 58 to 1530 of SEQ ID NO: 3; nucleotides 1 to 1479 of SEQ ID NO: 5; or nucleotides 1 to 1473 of SEQ ID NO: 7 and
   (ii) a cellulase and/or a hemicellulase and/or a pectinase,
      thereby to produce a sugar or sugars from a lignocellulosic material comprising a non-starch carbohydrate.

The methods of the invention as set out above may be carried out such that the polypeptide having peroxidase activity is contacted with the lignocellulosic non-starch carbohydrate material during, prior to, or subsequent to, a pre-treatment step (or may in fact itself be used as a pre-treatment step). Thus, the method of the invention may be an improved pre-treatment method since use of the polypeptide having peroxidase activity during, prior to or subsequent to pre-treatment may allow less chemical and/or energy to be used in such pre-treatment. Conditions for such pre-treatment may be more compatible with the conditions under which subsequent degradation of a non-starch carbohydrate take place.

The invention further provides a method for the preparation of a fermentation product, which method comprises:
a. oxidizing a lignocellulosic non-starch carbohydrate material or producing a sugar or sugars from a lignocellulosic non-starch carbohydrate material using a method as described above; and
b. fermenting the resulting material,
   thereby to prepare a fermentation product; and

### Brief description of the drawings

Figure 1 shows formation of brownish reaction products from guaiacol and MsP1 + H₂O₂ (1) compared to a heat inactivated reference + H₂O₂ (2).
Figure 2 shows HPLC-DAD (195nm) chromatogram of lignin (organosolv) extracts generated with active (-) or heat inactivated (- - -) MsP1 and H₂O₂.
Figure 3 shows HPLC-ELSD chromatogram of lignin (organosolv) extracts generated with active (-) or heat inactivated (- - -) MsP1 and *in situ* generation of H₂O₂.
Figure 4 shows laccase and peroxidase activities in submerged cultures of M. *scorodonius* (- Peroxidase ---- Laccase); medium A, -medium B•, medium C, medium D.
Figure 5 shows IEF electrophoresis with silver and ABTS staining, respectively. Lane 1: Reference proteins; lane 2-7 (silver stained) and 8-13 (ABTS staining): Medium A, days 2-7; lane 14-17 (ABTS staining): Medium A-D on day 11; lane 18; SNS medium on day 11.
Figure 6 shows induction of laccase and peroxidase activities in submerged cultures of *M. scorodonius*. SNS medium: Laccase (····), peroxidase (······); SNS supplemented with lignin: Laccase (~~~), peroxidase (^{······}); medium D supplemented with corn stover: Laccase (---), peroxidase (⁻)
Figure 7 shows β-glucosidase activity in submerged cultures of M. scorodonius supplemented with corn stover; medium A, -medium B-, medium C, medium D.
Figure 8 shows esterolytic activity in submerged cultures of M. *scorodonius.*
Figure 9 shows IEF electrophoresis with activity staining to visualise esterolytic activity; lane 1: medium B, day 7; lane 2: medium D, day 7; lane 3: medium B, day 10; lane 4: medium D, day 10; lane 5: medium B, day 14; lane 6: medium D, day 14.
Figure 10 shows release of glucose sugars from corn stover, expressed as percentage glucose of corn stover dm, as determined by reducing sugar assay.

### Brief description of the sequence listing

SEQ ID NO: 1 sets out the cDNA sequence of MsP1 from *Marasmius scorodonius.*

SEQ ID NO: 2 sets out the amino acid sequence of MsP1 from *Marasmuis scorodonius.* There is a signal peptide cleavage sequence at position 20/21. Accordingly, amino acids 21 to 513 represent the mature MsP1 sequence.

SEQ ID NO: 3 sets out the cDNA sequence of MsP2 from *Marasmius scorodonius.*

SEQ ID NO: 4 sets out the amino acid sequence of MsP2 from *Marasmius scorodonius.* There is a signal peptide cleavage sequence at position 19/20. Accordingly, amino acids 20 to 510 represent the mature MsP1 sequence.

SEQ ID NO: 5 sets out the nucleotide sequence used to express MsP1 from *Marasmius scorodonius* in *A. niger.*

SEQ ID NO: 6 sets out the amino acid sequence of MsP1 from *Marasmius scorodonius* expressed in *A. niger.*

SEQ ID NO: 7 sets out the nucleotide sequence used to express MsP2 from *Marasmius scorodonius* in *A. niger.*

SEQ ID NO: 8 sets out the amino acid sequence of MsP2 from *Marasmius scorodonius* expressed in *A. niger.*

### Detailed description of the invention

Throughout the present specification and the accompanying claims, the words "comprise" and "include" and variations such as "comprises", "comprising", "includes" and "including" are to be interpreted inclusively. That is, these words are intended to convey the possible inclusion of other elements or integers not specifically recited, where the context allows.

The articles "a" and "an" are used herein to refer to one or to more than one (i.e. to one or at least one) of the grammatical object of the article. By way of example, "an element" may mean one element or more than one element.

The present disclosure relates to the use of polypeptides having peroxidase activity. These polypeptides may be used to oxidizer a lignocellulosic material comprising a non-starch carbohydrate (also referred to herein as a non-starch comprising material). A lignocellulosic material which comprises a non-starch carbohydrate is one which comprises, consists of or substantially consists of one or more non-starch carbohydrates. Such a lignocellulosic non-starch carbohydrate may be a cellulose, a hemicellulose or a pectin/pectic substance.

Carbohydrate in this context includes all saccharides, for example polysaccharides, oligosaccharides, disaccharides or monosaccharides.

A polypeptide having peroxidase activity as used according to the disclosure may modify a lignocellulosic non-starch carbohydrate comprising material by chemically modifying or physically modifying such lignocellulosic material. Chemical modification of the lignocellulosic non-starch carbohydrate comprising material may result in the degradation of such material, for example by hydrolysis, oxidation or other chemical modification such as by the action of a lyase. Alternatively, modification may render the lignocellulosic non-starch carbohydrate comprising material more susceptible to enzymatic degradation, for example by an enzyme known to degrade a non-starch carbohydrate (eg. lignocellulose), such as a cellulase and/or a hemicellulase and/or a pectinase. A cellulase may comprise one or more of endocellulase, exocellulase or β-glucosidase activity. That is to say, the lignocellulosic non-starch carbohydrate comprising material may be modified such that the non-starch carbohydrate component of the material is rendered more susceptible to degradation by an enzyme that can degrade a non-starch carbohydrate.

Accordingly, the invention relates to a method for the oxidation of a lignocellulosic non-starch carbohydrate comprising material, which method comprises contacting said lignocellulosic non-starch carbohydrate material with a polypeptide having peroxidase activity as described herein.

Such oxidation may render the lignocellulosic material comprising a non-starch carbohydrate material more susceptible to enzymatic degradation (in particular render the non-starch carbohydrate component of the material more susceptible to enzymatic degradation). Accordingly, the invention relates to a method for increasing the susceptibility of a lignocellulosic non-starch carbohydrate comprising material to enzymatic degradation, which method comprises contacting said lignocellulosic non-starch carbohydrate comprising material with a polypeptide having peroxidase activity as described herein.

By "more susceptible to enzymatic degradation" is indicated that the lignocellulosic non-starch carbohydrate comprising material (in particular the non-starch carbohydrate component) will be degraded to a greater extent by an enzyme capable of carrying out such degradation when it is contacted with a peroxidase polypeptide as described herein as compared to the situation when it is not contacted with such a polypeptide.

A method of the invention may be a pre-treatment method. That is to say, a method of pre-treatment of a lignocellulosic material comprising a non-starch carbohydrate may be carried out which involves the use of a polypeptide having peroxidase activity. Thus, a method of the invention may be carried out such that the energy and/or chemical input during pre-treatment may be less than would otherwise be the case.

Thus, the invention provides a method for producing a sugar or sugars from a lignocellulosic material comprising a non-starch carbohydrate, which method comprises: contacting said lignocellulosic material comprising a non-starch carbohydrate with a peroxidase as described herein, i.e. oxidizing said lignocellulosic non-starch carbohydrate comprising material or increasing the susceptibility of such a lignocellulosic material to enzymatic degradation using a method as described herein; and contacting the thus modified lignocellulosic non-starch carbohydrate comprising material with a enzyme which degrades non-starch carbohydrate comprising material. Such an enzyme will typically be one which is capable of degrading the non-starch carbohydrate component of the material, such as a cellulase and/or a hemicellulase and/or a pectinase. In that way, one or more sugars may be produced from a lignocellulosic non-starch carbohydrate comprising material.

In the methods of the invention, a polypeptide used may be a polypeptide which has peroxidase activity, for example an isolated polypeptide, and which comprises a sequence according to any one of SEQ ID NOs: 2, 4, 6 or 8 or a functional equivalent or a fragment of such a polypeptide.

SEQ ID Nos: 6 and 8 set out the sequences of mature polypeptides (MsP1 and MsP2 respectively) having peroxidase activity from *Marasmius scorodonius.* Surprisingly, MsP1 and MsP2 are not comparable to known magnesium peroxidises and lignin peroxidases, but show more homology to the group of "DyP-type" peroxidases (dye decolorizing peroxidises). Also MsP1 and MsP2 occur as dimers. See Scheibner et al. Appl. Microbiol. Biotechnol. (2008) 77, 1241-1240, pp. 1247. SEQ ID NOs: 2 and 4, however, show full length sequences for MsP1 and MsP2 including signal sequences. There is a signal peptide cleavage site at position 20/21 of SEQ ID NO: 2 and at position 19/20 of SEQ ID NO: 4. Accordingly, a polypeptide used in the invention based on SEQ ID NO: 2 or 4 will typically comprise the sequence set out in amino acids 21 to 513 of SEQ ID NO: 2 or amino acids 20 to 510 of SEQ ID NO: 4 or be a functional equivalent or fragment of such a polypeptide.

A polypeptide for use in the invention, for example an isolated polypeptide, may be obtainable by expressing a polynucleotide comprising a sequence as set out in any one of SEQ ID NOs: 1, 3, 5 or 7 or a vector comprising a said polynucleotide in an appropriate host cell, e.g. *Aspergillus niger*. A polypeptide for use in the invention may be a functional equivalent or fragment of such a polypeptide.

In view of the fact that SEQ ID NOs: 1 and 3 comprise signal sequence encoding portions, a polypeptide of the disclosure may be encoded by a polynucleotide comprising a sequence as set out in nucleotides 61 to 1539 of SEQ ID NO: t or nucleotides 58 to 1530 of SEQ ID NO: 3. A polypeptide for use in the invention may be a functional equivalent or fragment of such a polypeptide.

The term 'polypeptide(s) having peroxidase activity' is here and hereafter defined as a polypeptide of EC 1.11.1.- which typically will be capable of catalysing a reaction of the form:

ROOR' + electron donor (2 e⁻) + 2H⁺ → ROH + R'OH

or

H₂A (electron donor) + H₂O₂ → 2H₂O + A

A polypeptide having peroxidase activity may have hydrogen peroxide as its optimal substrate, alternatively a polypeptide having peroxidase activity may be more active with an organic hydroperoxide such as a lipid peroxide. A polypeptide having peroxidase activity may contain a heme cofactor in its active site, or a redox-active cysteine or selenocysteine residue.

The method of the invention may make use of a purified polypeptide. The polypeptides described herein and suitable for use in the method of the invention include the polypeptides encoded by the polynucleotides described herein. Especially preferred is a polypeptide comprising the sequence set out in amino acids 21 to 513 of SEQ NO: 2; amino acids 20 to 510 of SEQ ID NO: 4; amino acids 1 to 493 of SEQ ID NO 6; or amino acids 1 to 491 of SEQ ID NO: 8 or a functional equivalent of any of those polypeptides.

Fusion proteins comprising a polypeptide described herein may also be used in the method of the invention.

Methods for making the polypeptides which may be used in the method of the invention are described herein.

Described herein are polynucleotides comprising a nucleotide sequence that hybridises, preferably under highly stringent conditions, to the reverse complement of a polynucleotide having the sequence according to any one of: nucleotides 61 to 1539 of SEQ ID NO: 1; nucleotides 58 to 1530 of SEQ ID NO: 3; nucleotides 1 to 1479 of SEQ ID NO: 5; or nucleotides 1 to 1473 of SEQ ID NO: 7.

Such nucleic acids, which may be used to provide polypeptides suitable for use in the invention, may comprise a sequence which has at least about 55%, preferably at least about 65%, more preferably at least about 70%, even more preferably at least about 75%, at least about 80%, at least about 85%, at least about 90%, at least about 95%, at least about 96%, at least about 97%, at least about 98% or at least about 99% sequence identity to a sequence as set out in any one of: nucleotides 61 to 1539 of SEQ ID NO: 1; nucleotides 58 to 1530 of SEQ ID NO: 3; nucleotides 1 to 1479 of SEQ ID NO: 5; or nucleotides 1 to 1473 of SEQ ID NO: 7.

In a more preferred embodiment, such a polynucleotide, or a polypeptide encoded by such a polypeptide, may be obtainable from a fungus, preferably a filamentous fungus, such as from the Dikarya, such as from the Basidiomycota, such as from the Agaricomycotina, such as from the Agaricomycetidae, such as from the Agaricales, such as from the Trichomataceae, in particular from the genus *Marasmius,* for example *Marasmius scorodonius.* A suitable polynucleotide may be obtained from a litter-decomposing fungus.

Such a polynucleotide may comprise a nucleic acid sequence encoding a polypeptide comprising an amino acid sequence as shown in any one of: amino acids 21 to 513 of SEQ NO: 2; amino acids 20 to 510 of SEQ ID NO: 4; amino acids 1 to 493 of SEQ ID NO 6; or amino acids 1 to 491 of SEQ ID NO: 8 or a functional equivalent or a fragment of such a polypeptide.

Such a polynucleotide may encode at least one functional domain of a polypeptide comprising the sequence set out in amino acids 21 to 513 of SEQ NO: 2; amino acids 20 to 510 of SEQ ID NO: 4; amino acids 1 to 493 of SEQ ID NO 6; or amino acids 1 to 491 of SEQ ID NO: 8 or at least one functional domain of a functional equivalent of such a polypeptide.

In a preferred embodiment, a method of the invention is carried out using a peroxidase enzyme encoded by a gene comprising the sequence according to any one of: nucleotides 61 to 1539 of SEQ ID NO: 1; nucleotides 58 to 1530 of SEQ ID NO: 3; nucleotides 1 to 1479 of SEQ ID NO: 5; or nucleotides 1 to 1473 of SEQ ID NO: 7. In another preferred embodiment, the invention is carried out where the peroxidase enzyme is encoded by a polynucleotide such that the amino acid sequence comprises any one of: amino acids 21 to 513 of SEQ NO: 2; amino acids 20 to 510 of SEQ ID NO: 4; amino acids 1 to 493 of SEQ ID NO 6; or amino acids 1 to 491 of SEQ ID NO: 8 or is a variant or a fragment of any of those polypeptides.

A polynucleotide giving rise to a polypeptide suitable for use in the invention may comprise the coding sequence coding for the polypeptides described herein, preferred is the polynucleotide sequence comprising any one of: nucleotides 61 to 1539 of SEQ ID NO: 1; nucleotides 58 to 1530 of SEQ ID NO: 3; nucleotides 1 to 1479 of SEQ ID NO: 5; or nucleotides 1 to 1473 of SEQ ID NO: 7.

The polynucleotides described herein may be incorporated into vectors in order to express a polypeptide which may be used in a method of the invention.

In such a vector, the polynucleotide sequence encoding a peroxidase may be functionally linked with regulatory sequences suitable for expression of the encoded amino acid sequence in a suitable host cell, such as a bacterium or a filamentous fungus, such as *Marasmius* for example *M. scorodonius* or *Aspergillus*, for example A. *niger* or *A. oryzae*.

A polypeptide suitable for use in the invention may be recombinantly produced in a host cell that contains a heterologous or homologous polynucleotide as described herein. In such a cell, the expression of a peroxidase may be significantly increased or activity of the peroxidase may be increased. Typically, such a cell is capable of producing a functional peroxidase according to the invention, preferably a cell capable of over-expressing the peroxidase, for example an *Aspergillus* strain comprising an increased copy number of a gene encoding such a peroxidase.

The method of the invention requires the use of an isolated polypeptide comprising the amino acid sequence set out in any one of: amino acids 21 to 513 of SEQ NO: 2; amino acids 20 to 510 of SEQ ID NO: 4; amino acids 1 to 493 of SEQ ID NO 6; or amino acids 1 to 491 of SEQ ID NO: 8 or an amino acid sequence obtainable by expressing a polynucleotide comprising the sequence set out in: nucleotides 61 to 1539 of SEQ ID NO: 1; nucleotides 58 to 1530 of SEQ ID NO: 3; nucleotides 1 to 1479 of SEQ ID NO: 5; or nucleotides 1 to 1473 of SEQ ID NO: 7, in an appropriate host. Also, a peptide or polypeptide comprising a functional equivalent of a fragment of such a polypeptide may be used in the present invention.

The terms "peptide" and "oligopeptide" are here and hereafter considered synonymous (as is commonly recognized) and each term can be used interchangeably as the context requires to indicate a chain of at least two amino acids coupled by peptidyl linkages. The word "polypeptide" is used herein for chains containing more than seven amino acid residues. All oligopeptide and polypeptide formulas or sequences herein are written from left to right and in the direction from amino terminus to carboxy terminus. The one-letter code of amino acids used herein is commonly known in the art and can be found in Sambrook, et al. (Molecular Cloning: A Laboratory Manual, 2nd, ed. Cold Spring Harbor Laboratory, Cold Spring Harbor Laboratory Press, Cold Spring Harbor, NY, 1989)

By "isolated" polypeptide or protein is intended a polypeptide or protein removed from its native environment. For example, recombinantly produced polypeptides and proteins expressed in host cells are considered isolated for the purpose of the invention as are native or recombinant polypeptides which have been substantially purified by any suitable technique such as, for example, the single-step purification method disclosed in Smith and Johnson, Gene 67:31-40 (1988).

The polypeptide described herein having peroxidase activity, or a functional equivalent or a fragment thereof, can be recovered and purified from recombinant cell cultures by well-known methods including ammonium sulfate or ethanol precipitation, acid extraction, anion or cation exchange chromatography, phosphocellulose chromatography, hydrophobic interaction chromatography, affinity chromatography, hydroxylapatite chromatography and lectin chromatography. Most preferably, high performance liquid chromatography ("HPLC") is employed for purification.

Polypeptides suitable for use in the present invention include naturally purified products, products of chemical synthetic procedures, and products produced by recombinant techniques from a prokaryotic or eukaryotic host, including, for example, bacterial, yeast, higher plant, insect and mammalian cells. Depending upon the host employed in a recombinant production procedure, the polypeptides of the present invention may be glycosylated or may be non-glycosylated. In addition, polypeptides may also include an initial modified methionine residue, in some cases as a result of host-mediated processes.

The terms "functional equivalent" and "functional variant" are used interchangeably herein. Functional equivalents of the peroxidase encoding polynucleotides described herein are isolated polynucleotides that encode a polypeptide that exhibits peroxidase activity, typically at least about the same or a better peroxidase activity of at least one of the peroxidase polypeptides defined herein (i.e. one comprising a sequence set out in any one of: amino acids 21 to 513 of SEQ NO: 2; amino acids 20 to 510 of SEQ ID NO: 4; amino acids 1 to 493 of SEQ ID NO 6; or amino acids 1 to 491 of SEQ ID NO: 8).

A functional equivalent of a peroxidase polypeptide is a polypeptide that exhibits peroxidase activity, typically at least the same or a better peroxidase activity of at least one of the peroxidase polypeptides defined herein (i.e. one comprising a sequence set out in any one of: amino acids 21 to 513 of SEQ NO: 2; amino acids 20 to 510 of SEQ ID NO: 4; amino acids 1 to 493 of SEQ ID NO 6; or amino acids 1 to 491 of SEQ ID NO: 8).

A functional protein or polypeptide equivalent may comprise one or more substitutions, insertions or deletions in comparison to a polypeptide comprising a sequence set out in any one of: amino acids 21 to 513 of SEQ NO: 2; amino acids 20 to 510 of SEQ ID NO: 4; amino acids 1 to 493 of SEQ ID NO 6; or amino acids 1 to 491 of SEQ ID NO: 8. A functional protein or polypeptide equivalent may contain only conservative substitutions of one or more amino acids in comparison to such a polypeptide. A substitution, insertion or deletion will typically be in respect of a non-essential amino acid.

A non-essential amino acid is a residue that can be altered in any one of the said sequences without substantially altering the biological function of the polypeptide. For example, amino acid residues that are conserved among the peroxidase proteins described herein, are predicted to be particularly unamenable to alteration. Furthermore, amino acids conserved among the peroxidase proteins described herein and other peroxidases are not likely to be amenable to alteration.

The term "conservative substitution" is intended to mean that a substitution in which the amino acid residue is replaced with an amino acid residue having a similar side chain. These families are known in the art and include amino acids with basic side chains (e.g., lysine, arginine and hystidine), acidic side chains (e.g. aspartic acid, glutamic acid), uncharged polar side chains (e.g., glycine, asparagines, glutamine, serine, threonine, tyrosine, cysteine), non-polar side chains (e.g., alanine, valine, leucine, isoleucine, proline, phenylalanine, methionine, tryptophan), beta-branched side chains (e.g., threonine, valine, isoleucine) and aromatic side chains (e.g., tyrosine, phenylalanine tryptophan, histidine).

A functional nucleic acid equivalent may typically contain mutations as compared with a polynucleotide comprising a sequence as set out in any one of: nucleotides 61 to 1539 of SEQ ID NO: 1; nucleotides 58 to 1530 of SEQ ID NO: 3; nucleotides 1 to 1479 of SEQ ID NO: 5; or nucleotides 1 to 1473 of SEQ ID NO: 7. Typically, such mutations will be silent mutations or mutations that do not alter the biological function of the encoded polypeptide.

Accordingly, nucleic acid molecules encoding a peroxidase protein polypeptide comprising a sequence set out in: amino acids 21 to 513 of SEQ NO: 2; amino acids 20 to 510 of SEQ ID NO: 4; amino acids 1 to 493 of SEQ ID NO 6; or amino acids 1 to 491 of SEQ ID NO: 8, that contain changes in amino acid residues that are not essential for a particular biological activity may be used to generate an enzyme for use in the invention.

A functional variant or functional equivalent peroxidase polypeptide will differ in amino acid sequence from any one of the sequences specifically described herein, yet will retain at least one biological activity of such a polypeptide.

In one embodiment an isolated nucleic acid molecule suitable for generating a polypeptide for use in the invention comprises a nucleotide sequence encoding a polypeptide, wherein the polypeptide comprises an amino acid sequence having at least about 55%, at least about 65%, at least about 70%, at least about 75%, at least about 80%, at least about 85%, at least about 90%, at least about 95%, at least about 96%, at least about 97%, at least about 98%, at least about 99% or more sequence identity to the amino acid sequence shown in any one of: amino acids 21 to 513 of SEQ NO: 2; amino acids 20 to 510 of SEQ ID NO: 4; amino acids 1 to 493 of SEQ ID NO 6; or amino acids 1 to 491 of SEQ ID NO: 8. Such a polypeptide may be a functional equivalent or functional variant suitable for use in the invention.

For example, guidance concerning how to make phenotypically silent amino acid substitutions is provided in Bowie, J.U. et al., Science 247:1306-1310 (1990) wherein the authors indicate that there are two main approaches for studying the tolerance of an amino acid sequence to change. The first method relies on the process of evolution, in which mutations are either accepted or rejected by natural selection. The second approach uses genetic engineering to introduce amino acid changes at specific positions of a cloned gene and selects or screens to identify sequences that maintain functionality. As the authors state, these studies have revealed that proteins are surprisingly tolerant of amino acid substitutions. The authors further indicate which changes are likely to be permissive at a certain position of the protein. For example, most buried amino acid residues require non-polar side chains, whereas few features of surface side chains are generally conserved. Other such phenotypically silent substitutions are described in Bowie *et al.*, supra, and the references cited therein.

An isolated nucleic acid molecule encoding an peroxidase protein as described herein can be created by introducing one or more nucleotide substitutions, additions or deletions into the coding sequences of a polynucleotide comprising any one of: nucleotides 61 to 1539 of SEQ ID NO: 1; nucleotides 58 to 1530 of SEQ ID NO: 3; nucleotides 1 to 1479 of SEQ ID NO: 5; or nucleotides 1 to 1473 of SEQ ID NO: 7, such that one or more amino acid substitutions, deletions or insertions are introduced into the encoded protein. Such mutations may be introduced by standard techniques, such as site-directed mutagenesis and PCR-mediated mutagenesis.

The term "functional equivalents" also encompasses orthologues of the M. *scorodonius* peroxidase MsP1 or Msp2 proteins as described herein. Orthologues of the *M. scorodonius* peroxidase MsP1 or Msp2 proteins are proteins that can be isolated from other strains or species and possess a similar or identical biological activity, in particular peroxidase activity. Such orthologues can readily be identified as comprising an amino acid sequence that is substantially homologous to any one of: amino acids 21 to 513 of SEQ NO: 2; amino acids 20 to 510 of SEQ ID NO: 4; amino acids 1 to 493 of SEQ ID NO 6; or amino acids 1 to 491 of SEQ ID NO: 8.

As defined herein, the term "substantially homologous" refers to a first amino acid or nucleotide sequence which contains a sufficient or minimum number of identical or equivalent (e.g., with similar side chain) amino acids or nucleotides to a second amino acid or nucleotide sequence such that the first and the second amino acid or nucleotide sequences have a common domain. For example, amino acid or nucleotide sequences which contain a common domain having at least about 55%, preferably at least about 65%, at least about 70%, more preferably at least about 75%, at least about 80%, at least about 85%, at least about 90%, at least about 95%, at least about 96%, at least about 97%, at least about 98% or at least about 99% identity or more are defined herein as sufficiently identical.

Also, nucleic acids encoding other peroxidase family members, which thus have a nucleotide sequence that differs from the sequences described herein, may be suitable for generating enzyme suitable for use in the invention. Moreover, nucleic acids encoding the peroxidase proteins described herein from different species which thus have a nucleotide sequence which differs from those described herein are also suitable for use in the invention.

Nucleic acid molecules corresponding to variants (e.g. natural allelic variants) and homologues of the peroxidase DNAs described herein can be isolated based on their homology to those nucleic acids, by using them or a suitable fragment thereof, as a hybridisation probe according to standard hybridisation techniques preferably under highly stringent hybridisation conditions.

In addition to naturally occurring allelic variants of the peroxidase sequences described herein, the skilled person will recognise that changes can be introduced by mutation into those nucleotide sequences thereby leading to changes in the amino acid sequence of the peroxidase protein without substantially altering the function of the protein.

Improved peroxidase proteins may be used in the invention. Improved peroxidase proteins are proteins wherein at least one biological activity, in particular peroxidase activity, is improved in comparison to a polypeptide comprising a sequence as set out in any one of: amino acids 21 to 513 of SEQ NO: 2; amino acids 20 to 510 of SEQ ID NO: 4; amino acids 1 to 493 of SEQ ID NO 6; or amino acids 1 to 491 of SEQ ID NO: 8. Such proteins may be obtained by randomly introducing mutations along all or part of sequences encoding such protein, such as by saturation mutagenesis, and the resulting mutants can be expressed recombinantly and screened for biological activity. For instance, the art provides for standard assays for measuring the enzymatic activity of peroxidases and thus improved proteins may easily be selected.

In a preferred embodiment the peroxidase protein suitable for use in the invention has an amino acid sequence according to any one of: amino acids 21 to 513 of SEQ NO: 2; amino acids 20 to 510 of SEQ ID NO: 4; amino acids 1 to 493 of SEQ ID NO 6; or amino acids 1 to 491 of SEQ ID NO: 8. In another embodiment, the peroxidase polypeptide is substantially homologous to such an amino acid sequence and retains at least one biological activity of such a polypeptide, for example peroxidase activity, yet differs in amino acid sequence due to natural variation or mutagenesis as described above.

In a further preferred embodiment, a peroxidase protein suitable for use in the invention comprises an amino acid sequence encoded by an nucleic acid fragment capable of hybridising to the reverse of complement of a nucleic acid sequence comprising any one of: nucleotides 61 to 1539 of SEQ ID NO: 1; nucleotides 58 to 1530 of SEQ ID NO: 3; nucleotides 1 to 1479 of SEQ ID NO: 5; or nucleotides 1 to 1473 of SEQ ID NO: 7, preferably under highly stringent hybridisation conditions.

Accordingly, the peroxidase polypeptide used in the methods described herein is a protein which comprises an amino acid sequence having least about 55%, at least about 65%, at least about 70%, at least about 75%, at least about 80%, at least about 85%, at least about 90%, at least about 95%, at least about 96%, at least about 97%, at least about 98%, at least about 99% or more sequence identity with the amino acid sequence of any one of: amino acids 21 to 513 of SEQ NO: 2; amino acids 20 to 510 of SEQ ID NO: 4; amino acids 1 to 493 of SEQ ID NO 6; or amino acids 1 to 491 of SEQ ID NO: 8 and which retains peroxidase activity.

A functional equivalent of a protein described herein can also be identified e.g. by screening combinatorial libraries of mutants, e.g. truncation mutants, of the protein of the invention for peroxidase activity. In one embodiment, a variegated library of variants is generated by combinatorial mutagenesis at the nucleic acid level. A variegated library of variants can be produced by, for example, enzymatically ligating a mixture of synthetic oligonucleotides into gene sequences such that a degenerate set of potential protein sequences is expressible as individual polypeptides, or alternatively, as a set of larger fusion proteins (e.g., for phage display). There are a variety of methods that can be used to produce libraries of potential variants of the polypeptides described herein from a degenerate oligonucleotide sequence. Methods for synthesizing degenerate oligonucleotides are known in the art (see, e.g., Narang (1983) Tetrahedron 39:3; Itakura et al. (1984) Annu. Rev. Biochem. 53:323; Itakura et al. (1984) Science 198:1056; Ike et al. (1983) Nucleic Acid Res. 11:477).

In addition, libraries of fragments of the coding sequence of a polypeptide described herein can be used to generate a variegated population of polypeptides for screening a subsequent selection of variants. For example, a library of coding sequence fragments can be generated by treating a double stranded PCR fragment of the coding sequence of interest with a nuclease under conditions wherein nicking occurs only about once per molecule, denaturing the double stranded DNA, renaturing the DNA to form double stranded DNA which can include sense/antisense pairs from different nicked products, removing single stranded portions from reformed duplexes by treatment with S1 nuclease, and ligating the resulting fragment library into an expression vector. By this method, an expression library can be derived which encodes N-terminal and internal fragments of various sizes of the protein of interest.

Several techniques are known in the art for screening gene products of combinatorial libraries made by point mutations of truncation, and for screening cDNA libraries for gene products having a selected property. The most widely used techniques, which are amenable to high through-put analysis, for screening large gene libraries typically include cloning the gene library into replicable expression vectors, transforming appropriate cells with the resulting library of vectors, and expressing the combinatorial genes under conditions in which detection of a desired activity facilitates isolation of the vector encoding the gene whose product was detected. Recursive ensemble mutagenesis (REM), a technique which enhances the frequency of functional mutants in the libraries, can be used in combination with the screening assays to identify variants of a protein of the invention (Arkin and Yourvan (1992) Proc. Natl. Acad. Sci. USA 89:7811-7815; Delgrave et al. (1993) Protein Engineering 6(3):327-331).

In addition to the peroxidase encoding sequences described herein, it will be apparent for the person skilled in the art that DNA sequence polymorphisms that may lead to changes in the amino acid sequence of the peroxidase-proteins may exist within a given population. Such genetic polymorphisms may exist in cells from different populations or within a population due to natural allelic variation. Allelic variants may also include functional equivalents.

A functional equivalent of a peroxidase encoding sequence suitable for use in the invention may be obtained using a labelled probe that comprises an isolated nucleic acid which encodes all or a portion of the sequence according to any one of: nucleotides 61 to 1539 of SEQ ID NO: 1; nucleotides 58 to 1530 of SEQ ID NO: 3; nucleotides 1 to 1479 of SEQ ID NO: 5; or nucleotides 1 to 1473 of SEQ ID NO: 7 or a variant of any of them; screening a nucleic acid fragment library with the labelled probe under conditions that allow hybridisation of the probe to nucleic acid fragments in the library, thereby forming nucleic acid duplexes, and preparing a full-length gene sequence from the nucleic acid fragments in any labelled duplex to obtain a gene related to a said coding sequence.

In one embodiment, a peroxidase polynucleotide useful for preparing a polypeptide for use in the invention may comprise a sequence having at least about 55%, at least about 65%, at least about 70%, at least about 75%, at least about 80%, at least about 85%, at least about 90%, at least about 91%, at least about 92%, at least about 93%, at least about 94%, at least about 95%, at least about 96%, at least about 97%, at least about 98%, at least about 99% or more sequence identity with any one of: nucleotides 61 to 1539 of SEQ ID NO: 1; nucleotides 58 to 1530 of SEQ ID NO: 3; nucleotides 1 to 1479 of SEQ ID NO: 5; or nucleotides 1 to 1473 of SEQ ID NO: 7 or the reverse complement any of them.

A peroxidase polypeptide suitable for use in the invention may comprise a sequence having at least about 55%, at least about 65%, at least about 70%, at least about 75%, at least about 80%, at least about 85%, at least about 90%, at least about 91%, at least about 92%, at least about 93%, at least about 94%, at least about 95%, at least about 96%, at least about 97%, at least about 98%, at least about 99% or more sequence identity with any one of amino acids 21 to 513 of SEQ NO: 2; amino acids 20 to 510 of SEQ ID NO: 4; amino acids 1 to 493 of SEQ ID NO 6; or amino acids 1 to 491 of SEQ ID NO: 8.

The present invention relates to methods in which polynucleotides encoding peroxidase enzymes are used which comprise an amino acid sequence according to any one of: amino acids 21 to 513 of SEQ NO: 2; amino acids 20 to 510 of SEQ ID NO: 4; amino acids 1 to 493 of SEQ ID NO 6; or amino acids 1 to 491 of SEQ ID NO: 8 or a functional equivalent or a fragment of such a polypeptide. The sequences having SEQ ID NO: 5 and SEQ ID NO: 7 (encoding peroxidases) are optimised versions of the genes as originally isolated, wherein a codon optimisation has taken place. Codon optimisation can be used according to methods known by the person skilled in the art and is especially suitable for improved expression of the gene in a host cell.

Also suitable for generating enzyme for use in the invention are polynucleotides comprising a sequence hybridisable under stringent conditions, preferably under highly stringent conditions, to the reverse complement of any one of: nucleotides 61 to 1539 of SEQ ID NO: 1; nucleotides 58 to 1530 of SEQ ID NO: 3; nucleotides 1 to 1479 of SEQ ID NO: 5; or nucleotides 1 to 1473 of SEQ ID NO: 7.

Advantageously, such polynucleotides may be obtained from fungi, preferably filamentous fungi, in particular from *Marasmius scorodonius.*

As used here and hereafter, the terms "gene" and "recombinant gene" refer to nucleic acid molecules which may be isolated from chromosomal DNA, which include an open reading frame encoding a protein, e.g. a *M. scorodonius* bleaching enzyme. A gene may include coding sequences, non-coding sequences, introns and regulatory sequences. Moreover, a gene refers to an isolated nucleic acid molecule as defined herein.

A nucleic acid molecule useful for generating a polypeptide suitable for use in a method of the present invention, such as one described herein, can be isolated using standard molecular biology techniques and the sequence information provided herein. For example, using all or a portion of the nucleic acid sequence comprising one of more of: nucleotides 61 to 1539 of SEQ ID NO: 1; nucleotides 58 to 1530 of SEQ ID NO: 3; nucleotides 1 to 1479 of SEQ ID NO: 5; or nucleotides 1 to 1473 of SEQ ID NO: 7 as a hybridization probe, nucleic acid molecules according to the invention can be isolated using standard hybridization and cloning techniques (e. g., as described in Sambrook, J., Fritsh, E. F., and Maniatis, T. Molecular Cloning: A Laboratory Manual.2nd, ed., Cold Spring Harbor Laboratory, Cold Spring Harbor Laboratory Press, Cold Spring Harbor, NY, 1989).

Moreover, a nucleic acid molecule encompassing all or a portion of any one of: nucleotides 61 to 1539 of SEQ ID NO: 1; nucleotides 58 to 1530 of SEQ ID NO: 3; nucleotides 1 to 1479 of SEQ ID NO: 5; or nucleotides 1 to 1473 of SEQ ID NO: 7 can be isolated by the polymerase chain reaction (PCR) using synthetic oligonucleotide primers designed based upon that sequence information.

Suitable nucleic acid of the invention can be amplified using cDNA, mRNA or alternatively, genomic DNA, as a template and appropriate oligonucleotide primers according to standard PCR amplification techniques. The nucleic acid so amplified can be cloned into an appropriate vector and characterized by DNA sequence analysis.

Furthermore, oligonucleotides corresponding to or hybridisable to nucleotide sequences described herein can be prepared by standard synthetic techniques, e.g. using an automated DNA synthesizer.

A nucleic acid molecule useful for generating a polypeptide suitable for use in a method of the invention may comprise a nucleic acid molecule which is the reverse complement of the nucleotide sequence described herein or a functional equivalent of these nucleotide sequences.

A nucleic acid molecule which is complementary to another nucleotide sequence is one which is sufficiently complementary to the other nucleotide sequence such that it can hybridize to the other nucleotide sequence thereby forming a stable duplex.

One aspect of the disclosure pertains to nucleic acid molecules that encode a polypeptide suitable for use in the method of the invention or a functional equivalent thereof such as a biologically active fragment or domain, as well as nucleic acid molecules sufficient for use as hybridisation probes to identify nucleic acid molecules encoding a polypeptide of the invention and fragments of such nucleic acid molecules suitable for use as PCR primers for the amplification or mutation of nucleic acid molecules.

An "isolated polynucleotide" or "isolated nucleic acid" is a DNA or RNA that is not immediately contiguous with both of the coding sequences with which it is immediately contiguous (one on the 5' end and one on the 3' end) in the naturally occurring genome of the organism from which it is derived. Thus, a nucleic acid may includes some or all of the 5' non-coding (e.g., promotor) sequences that are immediately contiguous to the coding sequence. The term therefore includes, for example, a recombinant DNA that is incorporated into a vector, into an autonomously replicating plasmid or virus, or into the genomic DNA of a prokaryote or eukaryote, or which exists as a separate molecule (e.g., a cDNA or a genomic DNA fragment produced by PCR or restriction endonuclease treatment) independent of other sequences. It also includes a recombinant DNA that is part of a hybrid gene encoding an additional polypeptide that is substantially free of cellular material, viral material, or culture medium (when produced by recombinant DNA techniques), or chemical precursors or other chemicals (when chemically synthesized). Moreover, an "isolated nucleic acid fragment" is a nucleic acid fragment that is not naturally occurring as a fragment and would not be found in the natural state.

As used herein, the terms "polynucleotide" or "nucleic acid molecule" are intended to include DNA molecules (e.g., cDNA or genomic DNA) and RNA molecules (e.g., mRNA) and analogs of the DNA or RNA generated using nucleotide analogs. The nucleic acid molecule can be single-stranded or double-stranded, but preferably is double-stranded DNA. The nucleic acid may be synthesized using oligonucleotide analogs or derivatives (e.g., inosine or phosphorothioate nucleotides). Such oligonucleotides can be used, for example, to prepare nucleic acids that have altered base-pairing abilities or increased resistance to nucleases.

The sequence information as provided herein should not be so narrowly construed as to require inclusion of erroneously identified bases. The specific sequences disclosed herein can be readily used to isolate the complete gene from fungi, in particular *M*. *scorodonius* which in turn can easily be subjected to further sequence analyses thereby identifying sequencing errors.

Unless otherwise indicated, all nucleotide sequences determined by sequencing a DNA molecule herein were determined using an automated DNA sequencer and all amino acid sequences of polypeptides encoded by DNA molecules determined herein were predicted by translation of a DNA sequence determined as above. Therefore, as is known in the art for any DNA sequence determined by this automated approach, any nucleotide sequence determined herein may contain some errors. Nucleotide sequences determined by automation are typically at least about 90% identical, more typically at least about 95% to at least about 99.9% identical to the actual nucleotide sequence of the sequenced DNA molecule. The actual sequence can be more precisely determined by other approaches including manual DNA sequencing methods well known in the art. As is also known in the art, a single insertion or deletion in a determined nucleotide sequence compared to the actual sequence will cause a frame shift in translation of the nucleotide sequence such that the predicted amino acid sequence encoded by a determined nucleotide sequence will be completely different from the amino acid sequence actually encoded by the sequenced DNA molecule, beginning at the point of such an insertion or deletion.

The person skilled in the art is capable of identifying such erroneously identified bases and knows how to correct for such errors.

The terms "homology" or "percent(age) identity" or "sequence identity" and the like are used interchangeably herein. For the purpose of this invention, it is defined here that in order to determine the degree of sequence identity shared by two amino acid sequences or of two nucleic acid sequences, the sequences are aligned for optimal comparison purposes (e.g., gaps can be introduced in the sequence of a first amino acid or nucleic acid sequence for optimal alignment with a second amino or nucleic acid sequence). Such alignment may be carried out over the full lengths of the sequences being compared. Alternatively, the alignment may be carried out over a shorter length, for example over about 20, about 50, about 100 or more nucleic acids/based or amino acids. The amino acid residues or nucleotides at corresponding amino acid positions or nucleotide positions are then compared. When a position in the first sequence is occupied by the same amino acid residue or nucleotide as the corresponding position in the second sequence, then the molecules are identical at that position. The percent identity between the two sequences is a function of the number of identical positions shared by the sequences (i.e., % identity = number of identical positions/total number of positions (i.e. overlapping positions) x 100). Preferably, the two sequences are the same length. The two sequences may be aligned over their entire lengths.

The skilled person will be aware of the fact that several different computer programs are available to determine the homology between two sequences. For instance, a comparison of sequences and determination of percent identity between two sequences can be accomplished using a mathematical algorithm.

In a preferred embodiment, the sequence identity between two amino acid sequences is determined using the Needleman and Wunsch (J. Mol. Biol. (48):444-453 (1970)) algorithm which has been incorporated into the GAP program in the GCG software package (available athttp://www.accelrys.com/solutions/bioinformatician/), using either a Blossom 62 matrix or a PAM250 matrix, and a gap weight of 16, 14, 12, 10, 8, 6, or 4 and a length weight of 1, 2, 3, 4, 5, or 6. The skilled person will appreciate that all these different parameters will yield slightly different results but that the overall percentage identity of two sequences is not significantly altered when using different algorithms.

In another embodiment, the sequence identity between two nucleotide sequences is determined using the GAP program in the GCG software package, using a NWSgapdna.CMP matrix and a gap weight of 40, 50, 60, 70, or 80 and a length weight of 1, 2, 3, 4, 5, or 6. In another embodiment, the percent identity two amino acid or nucleotide sequence is determined using the algorithm of E. Meyers and W. Miller (CABIOS, 4:11-17 (1989) which has been incorporated into the ALIGN program (version 2.0) (available at: http://vega.igh.cnrs.fr/bin/align-guess.cgi) using a PAM120 weight residue table, a gap length penalty of 12 and a gap penalty of 4.

Alternatively, the sequence identity between two amino acid or nucleotide sequence may be determined using, for example, the algorithm of E. Meyers and W. Miller (CABIOS, 4:11-17 (1989) which has been incorporated into the ALIGN program (version 2.0) (available at the ALIGN Query using sequence data of the Genestream server IGH Montpellier France http://vega.igh.cnrs.fr/bin/align-guess.cgi) using a PAM120 weight residue table, a gap length penalty of 12 and a gap penalty of 4.

The nucleic acid and protein sequences of the present disclosure can further be used as a "query sequence" to perform a search against public databases to, for example, identify other family members or related sequences. Such searches can be performed using the BLASTN, BLASTP and BLASTX programs (version 2.0) of Altschul, et al. (1990) J. Mol. Biol. 215:403-10. BLAST nucleotide searches in the nucleotide databases can be performed with the BLASTN program to obtain nucleotide sequences homologous to ZFX nucleic acid molecules of the invention. BLAST searches with a translated nucleotide sequence in the protein databases can be performed with the BLASTX program to obtain amino acid sequences homologous to the translated ZFX gene of the invention. Alternatively, for protein sequence comparison with the protein databases the BLASP program can be used with matrix Blosum 62, an expected threshold = 10, word length = 3, gap existence costs of 11 and gap extension costs of 1. When utilizing BLAST programs, the default parameters of the respective programs (e.g., BLASTX, BLASTP and BLASTN) can be used. See the homepage of the National Center for Biotechnology Information at http://www.ncbi.nlm.nih.gov/ for all relevant information on homology searches in public databases.

The BLASTP and BLAST N algorithms can be used to calculate sequence identity or to line up sequences (such as identifying equivalent or corresponding sequences, for example on their default settings).

Software for performing BLAST analyses is publicly available through the National Center for Biotechnology Information (http://www.ncbi.nlm.nih.gov/). This algorithm involves first identifying high scoring sequence pair (HSPs) by identifying short words of length W in the query sequence that either match or satisfy some positive-valued threshold score T when aligned with a word of the same length in a database sequence. T is referred to as the neighborhood word score threshold. These initial neighborhood word hits act as seeds for initiating searches to find HSPs containing them. The word hits are extended in both directions along each sequence for as far as the cumulative alignment score can be increased. Extensions for the word hits in each direction are halted when: the cumulative alignment score falls off by the quantity X from its maximum achieved value; the cumulative score goes to zero or below, due to the accumulation of one or more negative-scoring residue alignments; or the end of either sequence is reached. The BLAST algorithm parameters W, T and X determine the sensitivity and speed of the alignment. The BLASTN program from DNA-DNA comparison uses as defaults a word length (W) of 11, expectation (E) of 10, and a comparison of both strands. The BLASTP program for protein-protein comparison uses as defaults a word length (W) of 3, the BLOSUM62 scoring matrix, a gap existence penalty of 11 with a gap extension penalty of 1, and an expectation (E) of 10.

The BLAST algorithm performs a statistical analysis of the similarity between two sequences. One measure of similarity provided by the BLAST algorithm is the smallest sum probability (P(N)), which provides an indication of the probability by which a match between two nucleotide or amino acid sequences would occur by chance. For example, a sequence is considered similar to another sequence if the smallest sum probability in comparison of the first sequence to the second sequence is less than about 1, preferably less than about 0.1, more preferably less than about 0.01, and most preferably less than about 0.001.

Also, a peptide motif can be used to identify genes that code for proteins containing this peptide motif. Instead of one peptide motif, also a combination of two or more peptide motifs can be used to identify genes coding for proteins containing the peptide motifs. When one or several peptide motifs coding for specific proline-specific proteases are identified it is thus possible to identify genes coding for proline-specific proteases using one or a combination of several of these peptide motifs. Proline-specific proteases are used as an example how such genes may be identified, but the methods described are generally applicable. A peptide motif can be used for a search in translated DNA sequences from a DNA databank or protein sequences from a protein sequence databank using a program like Patscan (http://www-unix.mcs.anl.gov/compbio/PatScan/HTML/). The amino acid sequence has to be entered in a special format that is described on the website. Another method that can be performed is to use the sequence of the motif for a search in translated DNA sequences from a DNA databank or protein sequences from a protein sequence databank using a program like http://myhits.isb-sib.ch/cgi-bin/. For this program the motif is entered in the search field in the so called Prosite format, and databases are searched for the presence of the motif in the protein sequence or in the translated DNA sequence. This method can be used to identify fungal genes that encode useful proline-specific proteases. The genes that are identified using one of these methods can than be translated into a protein sequence using programs known to those skilled in the art, and be inspected for the presence of a signal sequence at their amino-terminus. For detecting a signal sequence one can use a program like SignalP (http://www.cbs.dtu.dk/services/SignalP/). Looking for a protein sequence that contains both the consensus sequences and a predicted signal sequence gives a large advantage for the industrial production of such an enzyme.

Sequences, nucleotide and polypeptide, identified in this way may be suitable for use in a method of the invention.

As used herein, the term "hybridizing" is intended to describe conditions for hybridization and washing under which nucleotide sequences having at least about 55%, at least about 40%, at least about 70%, more preferably at least about 80%, even more preferably at least about 85%, at least about 90%, more preferably at least about 95%, at least about 98%, at least about 99% sequence identity with each other typically remain hybridized to each other.

A preferred, non-limiting example of such hybridization conditions are hybridization in 6X sodium chloride/sodium citrate (SSC) at about 45 °C, followed by one or more washes in 1 X SSC, 0.1 % SDS at about 50 °C, preferably at about 55 °C, preferably at about 60 °C and even more preferably at about 65 °C.

Highly stringent conditions include, for example, hybridizing at about 68 °C in 5x SSC/5x Denhardt's solution / 1.0% SDS and washing in 0.2x SSC/0.1% SDS at about room temperature. Alternatively, washing may be performed at 42 °C.

The skilled artisan will know which conditions to apply for stringent and highly stringent hybridisation conditions. Additional guidance regarding such conditions is readily available in the art, for example, in Sambrook et al., 1989, Molecular Cloning, A Laboratory Manual, Cold Spring Harbor Press, N.Y.; and Ausubel et al. (eds.), 1995, Current Protocols in Molecular Biology, (John Wiley & Sons, N.Y.).

Of course, a polynucleotide which hybridizes only to a poly A sequence (such as the 3' terminal poly(A) tract of mRNAs), or to a complementary stretch of T (or U) resides, would not be included in a polynucleotide of the invention used to specifically hybridize to a portion of a nucleic acid of the invention, since such a polynucleotide would hybridize to any nucleic acid molecule containing a poly (A) stretch or the complement thereof (e.g., practically any double-stranded cDNA clone).

In a typical approach, cDNA libraries constructed from other organisms, e.g. filamentous fungi, in particular from the species *Marasmius* can be screened to obtain further polynucleotides encoding polypeptides which may be used in the invention.

For example, *Marasmius* strains can be screened for homologous peroxidase polynucleotides by Northern blot analysis. Upon detection of transcripts homologous to polynucleotides according to the invention, cDNA libraries can be constructed from RNA isolated from the appropriate strain, utilizing standard techniques well known to those of skill in the art. Alternatively, a total genomic DNA library can be screened using a probe hybridisable to a peroxidase polynucleotide as described herein.

Homologous gene sequences can be isolated, for example, by performing PCR using two degenerate oligonucleotide primer pools designed on the basis of nucleotide sequences as taught herein.

The template for the reaction can be cDNA obtained by reverse transcription of mRNA prepared from strains known or suspected to express a polynucleotide according to the invention. The PCR product can be subcloned and sequenced to ensure that the amplified sequences represent the sequences of a new peroxidase nucleic acid sequence, or a functional equivalent thereof.

The PCR fragment can then be used to isolate a full-length cDNA clone by a variety of known methods. For example, the amplified fragment can be labelled and used to screen a bacteriophage or cosmid cDNA library. Alternatively, the labelled fragment can be used to screen a genomic library.

PCR technology also can be used to isolate full-length cDNA sequences from other organisms. For example, RNA can be isolated, following standard procedures, from an appropriate cellular or tissue source. A reverse transcription reaction can be performed on the RNA using an oligonucleotide primer specific for the most 5' end of the amplified fragment for the priming of first strand synthesis.

The resulting RNA/DNA hybrid can then be "tailed" (e.g., with guanines) using a standard terminal transferase reaction, the hybrid can be digested with RNase H, and second strand synthesis can then be primed (e.g., with a poly-C primer). Thus, cDNA sequences upstream of the amplified fragment can easily be isolated. For a review of useful cloning strategies, see e.g., Sambrook *et al.,* supra; and Ausubel *et al.,* supra.

A vector, preferably an expression vector, may contain a nucleic acid encoding a peroxidase protein as defined herein or a functional equivalent thereof. As used herein, the term "vector" refers to a nucleic acid molecule capable of transporting another nucleic acid to which it has been linked. One type of vector is a "plasmid", which refers to a circular double stranded DNA loop into which additional DNA segments can be ligated. Another type of vector is a viral vector, wherein additional DNA segments can be ligated into the viral genome. Certain vectors are capable of autonomous replication in a host cell into which they are introduced (e.g., bacterial vectors having a bacterial origin of replication and episomal mammalian vectors). Other vectors (e.g., non-episomal mammalian vectors) are integrated into the genome of a host cell upon introduction into the host cell, and thereby are replicated along with the host genome. Moreover, certain vectors are capable of directing the expression of genes to which they are operatively linked. Such vectors are referred to herein as "expression vectors". In general, expression vectors of utility in recombinant DNA techniques are often in the form of plasmids. The terms "plasmid" and "vector" can be used interchangeably herein as the plasmid is the most commonly used form of vector. However, method of the invention is intended to include such other forms of expression vectors, such as viral vectors (e.g., replication defective retroviruses, adenoviruses and adeno-associated viruses), which serve equivalent functions.

The recombinant expression vectors described herein comprise a nucleic acid in a form suitable for expression of the nucleic acid in a host cell, which means that the recombinant expression vector includes one or more regulatory sequences, selected on the basis of the host cells to be used for expression, which is operatively linked to the nucleic acid sequence to be expressed. Within a recombinant expression vector, "operatively linked" is intended to mean that the nucleotide sequence of interest is linked to the regulatory sequence(s) in a manner which allows for expression of the nucleotide sequence (e.g., in an *in vitro* transcription/translation system or in a host cell when the vector is introduced into the host cell). The term "regulatory sequence" is intended to include promoters, enhancers and other expression control elements (e.g., polyadenylation signal). Such regulatory sequences are described, for example, in Goeddel; Gene Expression Technology: Methods in Enzymology 185, Academic Press, San Diego, CA (1990). Regulatory sequences include those which direct constitutive expression of a nucleotide sequence in many types of host cells and those which direct expression of the nucleotide sequence only in a certain host cell (e.g. tissue-specific regulatory sequences). It will be appreciated by those skilled in the art that the design of the expression vector can depend on such factors as the choice of the host cell to be transformed, the level of expression of protein desired, etc. Such expression vectors can be introduced into host cells to thereby produce proteins or peptides, encoded by nucleic acids as described herein.

The recombinant expression vectors can be designed for expression of peroxidase proteins in prokaryotic or eukaryotic cells. For example, peroxidase proteins can be expressed in fungal cells, bacterial cells such as *E. coli*, insect cells (using baculovirus expression vectors) yeast cells or mammalian cells. Suitable host cells are discussed further in Goeddel, Gene Expression Technology: Methods in Enzymology 185, Academic Press, San Diego, CA (1990). Alternatively, the recombinant expression vector can be transcribed and translated *in vitro*, for example using T7 promoter regulatory sequences and T7 polymerase.

Expression vectors which may be used to generate polypeptide useful in the present invention include chromosomal-, episomal- and virus-derived vectors e.g., vectors derived from bacterial plasmids, bacteriophage, yeast episome, yeast chromosomal elements, viruses such as baculoviruses, papova viruses, vaccinia viruses, adenoviruses, fowl pox viruses, pseudorabies viruses and retroviruses, and vectors derived from combinations thereof, such as those derived from plasmid and bacteriophage genetic elements, such as cosmids and phagemids.

The DNA insert should be operatively linked to an appropriate promoter, such as the phage lambda PL promoter, the *E*. *coli* lac, trp and tac promoters, the SV40 early and late promoters and promoters of retroviral LTRs, to name a few. Other suitable promoters will be known to the skilled person. In a specific embodiment, promoters are preferred that are capable of directing a high expression level of peroxidases in prokaryotes or filamentous fungi. Such promoters are known in the art. The expression constructs may contain sites for transcription initiation, termination, and, in the transcribed region, a ribosome binding site for translation. The coding portion of the mature transcripts expressed by the constructs will include a translation initiating AUG at the beginning and a termination codon appropriately positioned at the end of the polypeptide to be translated.

Vector DNA can be introduced into prokaryotic or eukaryotic cells via conventional transformation or transfection techniques. As used herein, the terms "transformation" and "transfection" are intended to refer to a variety of art-recognized techniques for introducing foreign nucleic acid (e.g., DNA) into a host cell, including calcium phosphate or calcium chloride co-percipitation, DEAE-dextran-mediated transfection, transduction, infection, lipofection, cationic lipidmediated transfection or electroporation. Suitable methods for transforming or transfecting host cells can be found in Sambrook, et al. (Molecular Cloning: A Laboratory Manual, 2nd,ed. Cold Spring Harbor Laboratory, Cold Spring Harbor Laboratory Press, Cold Spring Harbor, NY, 1989), Davis et al., Basic Methods in Molecular Biology (1986) and other laboratory manuals.

For stable transfection of mammalian cells, it is known that, depending upon the expression vector and transfection technique used, only a small fraction of cells may integrate the foreign DNA into their genome. In order to identify and select these integrants, a gene that encodes a selectable marker (e.g., resistance to antibiotics) is generally introduced into the host cells along with the gene of interest. Preferred selectable markers include those which confer resistance to drugs, such as G418, hygromycin and methatrexate. Nucleic acid encoding a selectable marker can be introduced into a host cell on the same vector as that encoding a peroxidase polypeptide or can be introduced on a separate vector. Cells stably transfected with the introduced nucleic acid can be identified by drug selection (e.g. cells that have incorporated the selectable marker gene will survive, while the other cells die).

Expression of proteins in prokaryotes is often carried out in *E*. *coli* with vectors containing constitutive or inducible promoters directing the expression of either fusion or non-fusion proteins. Fusion vectors add a number of amino acids to a protein encoded therein, e.g. to the amino terminus of the recombinant protein. Such fusion vectors typically serve three purposes: 1) to increase expression of recombinant protein; 2) to increase the solubility of the recombinant protein; and 3) to aid in the purification of the recombinant protein by acting as a ligand in affinity purification. Often, in fusion expression vectors, a proteolytic cleavage site is introduced at the junction of the fusion moiety and the recombinant protein to enable separation of the recombinant protein from the fusion moiety subsequent to purification of the fusion protein. Such enzymes, and their cognate recognation sequences, include Factor Xa, thrombin and enterokinase.

As indicated, the expression vectors will preferably contain selectable markers. Such markers include dihydrofolate reductase or neomycin resistance for eukarotic cell culture and tetracyline or ampicillin resistance for culturing in *E*. *coli* and other bacteria. Representative examples of appropriate host include bacterial cells, such as *E*. *coli*, Streptomyces and Salmonella typhimurium; fungal cells, such as yeast; insect cells such as Drosophila S2 and Spodoptera Sf9; animal cells such as CHO, COS and Bowes melanoma; and plant cells. Appropriate culture media and conditions for the above-described host cells are known in the art.

Among vectors preferred for use in bacteria are pQE70, pQE60 and PQE-9, available from Qiagen; pBS vectors, Phagescript vectors, Bluescript vectors, pNH8A, pNH16A, pNH18A, pNH46A, available from Stratagene; and ptrc99a, pKK223-3, pKK233-3, pDR540, pRIT5 available from Pharmacia. Among preferred eukaryotic vectors are PWLNEO, pSV2CAT, pOG44, pZT1 and pSG available from Stratagene; and pSVK3, pBPV, pMSG and pSVL available from Pharmacia. Other suitable vectors will be readily apparent to the skilled artisan.

Among known bacterial promotors for use in the present invention include *E. coli* lacl and lacZ promoters, the T3 and T7 promoters, the gpt promoter, the lambda PR, PL promoters and the trp promoter, the HSV thymidine kinase promoter, the early and late SV40 promoters, the promoters of retroviral LTRs, such as those of the Rous sarcoma virus ("RSV"), and metallothionein promoters, such as the mouse metallothionein-I promoter.

Transcription of the DNA encoding the polypeptides of the present invention by higher eukaryotes may be increased by inserting an enhancer sequence into the vector. Enhancers are cis-acting elements of DNA, usually about from 10 to 300 bp that act to increase transcriptional activity of a promoter in a given host cell-type. Examples of enhancers include the SV40 enhancer, which is located on the late side of the replication origin at bp 100 to 270, the cytomegalovirus early promoter enhancer, the polyoma enhancer on the late side of the replication origin, and adenovirus enhancers.

For secretion of the translated protein into the lumen of the endoplasmic reticulum, into the periplasmic space or into the extracellular environment, appropriate secretation signal may be incorporated into the expressed polypeptide. The signals may be endogenous to the polypeptide or they may be heterologous signals.

The polypeptide may be expressed in a modified form, such as a fusion protein, and may include not only secretion signals but also additional heterologous functional regions. Thus, for instance, a region of additional amino acids, particularly charged amino acids, may be added to the N-terminus of the polypeptide to improve stability and persistence in the host cell, during purification or during subsequent handling and storage. Also, peptide moieties may be added to the polypeptide to facilitate purification.

Also described herein are cells, e.g., transformed host cells or recombinant host cells that contain a nucleic acid which encodes a polypeptide suitable for use in the method of the invention. A "transformed cell" or "recombinant cell" is a cell into which (or into an ancestor of which) has been introduced, by means of recombinant DNA techniques, a nucleic acid as described herein. Both prokaryotic and eukaryotic cells are included, e.g., bacteria, fungi, yeast, and the like.

Examples of suitable host cells are *Microcystis, Lepista,* for example *L. irina, Cyathus,* for example *C. pallidus*, *Ganoderma,* for example *G*. *applanatum*, *Ischnoderma,* for example *I. benzoinum, Marasmius,* for example *M*. *scorodonius, Trametes,* for example *T. suaveolens* of *T. versicolor*, *Cryptococcus,* for example *C*. *laurentii*, *Hypomyces,* for example *H. odoratus* or *Phaffia*, for example *P. rhodozyma, Phanerochaete* for example *P. chrysosporium*, *Lentinula* for example *L. edodes, Coprinus* for example *C*. *cinereus*, *Gloeophyllum* for example *G. trabeum, Ophiostoma* for example *O. piliferum*, *Aspergillus* for example *A. niger, A. oryzae*, *A. nidulans*, *Thermomyces,* for example *T. lanuginosa, Sporotrichum,* for example *S*. *thermophile, Aureobasidium* for example *A. pullulans, Amorphotheca,* for example *A. resinae, Leucosporidium,* for example *L. scottii, Cunninghamella,* for example *C*. *elegans.*

Especially preferred are cells from filamentous fungi, in particular *Aspergillus -* for example *Aspergillus oryzae* or *Aspergillus niger* - and *Marasmius* - for example *Marasmius scorodonius -* or cells from yeasts such as *Pichia*, - for example *Pichia Pastoris* - or cells from bacteria.

A host cell can be chosen that modulates the expression of the inserted sequences, or modifies and processes the gene product in a specific, desired fashion. Such modifications (e.g. glycosylation) and processing (e.g. cleavage) of protein products may facilitate optimal functioning of the protein.

Various host cells have characteristic and specific mechanisms for post-translational processing and modification of proteins and gene products. Appropriate cell lines or host systems familiar to those of skill in the art of molecular biology and/or microbiology can be chosen to ensure the desired and correct modification and processing of the foreign protein expressed. To this end, eukaryotic host cells that possess the cellular machinery for proper processing of the primary transcript, glycosylation, and phosphorylation of the gene product can be used. Such host cells are well known in the art.

Host cells also include, but are not limited to, mammalian cell lines such as CHO, VERO, BHK, HeLa, COS, MDCK, 293, 3T3, WI38, and choroid plexus cell lines.
If desired, the polypeptides can be produced by a stably-transfected cell line. A number of vectors suitable for stable transfection of mammalian cells are available to the public, methods for constructing such cell lines are also publicly known, e.g., in Ausubel *et al.* (supra).

In the method of the invention, a polypeptide is contacted with a material comprising a non-starch carbohydrate.

The polypeptide may be added as isolated or purified enzyme, an enzyme preparation or produced *in situ* by a microorganism capable of producing said enzyme. The enzyme preparation can be derived from various sources, for example from plants, animals and microorganisms. Preferably the enzyme preparation is derived from a microorganism, since microorganisms make it possible to obtain the enzyme on an industrial scale in a controlled manner. The enzyme preparation derived from a microorganism can be obtained by classical fermentation processes of a selected microbial strain or by fermentation of a microorganism that over expresses the enzyme. The microorganism may be a bacterium, a fungus or yeast. Examples of suitable microorganisms are *Microcystis, Lepista,* for example *L. irina, Cyathus,* for example *C*. *pallidus*, *Ganoderma,* for example *G. applanatum, Ischnoderma,* for example *I. benzoinum*, *Marasmius*, for example *M. scorodonius, Trametes,* for example *T*. *suaveoluens* of *T. versicolour, Cryptococcus,* for example *C*. *laurentii*, *Hypomyces,* for example *H. odoratus* or *Phaffia*, for example *P. rhodozyma, Phanerochaete* for example *P. chrysosporium*, *Lentinula* for example *L. edodes, Coprinus* for example *C*. *cinereus, Gloeophyllum* for example *G*. *trabeum*, *Ophiostoma* for example *O. piliferum, Aspergillus* for example *A. niger, A. oryzae*, *A. nidulans*, *Thermomyces,* for example *T. lanuginosa, Sporotrichum,* for example *S*. *thermophile, Aureobasidium* for example *A. pullulans, Amorphotheca,* for example *A. resinae, Leucosporidium,* for example *L*. *scottii, Cunninghamella,* for example *C*. *elegans.*

The methods of the invention may be carried out wherein the polypeptide is added as an enzyme preparation derived from or produced *in situ* by a plant, for example a corn plant, a rice plant, a sugar cane plant, a wheat plant or a barley plant.

A lignocellulosic material comprising a non-starch carbohydrate suitable for oxidation by a polypeptide is typically lignocellulose. The major polysaccharides comprising different lignocellulosic residues, which may be considered as a potential renewable feedstock, are cellulose (glucans), hemicelluloses (xylans, heteroxylans and xyloglucans). In addition, some hemicellulose may be present as glucomannans, for example in wood-derived feedstocks. The enzymatic hydrolysis of these polysaccharides to soluble sugars, for example glucose, xylose, arabinose, galactose, sucrose, fructose, mannose, rhamnose, ribose, D-galacturonic acid and other hexoses and pentoses occurs under the action of different enzymes acting in concert.

In addition, pectins and other pectic substances such as arabinans may make up considerably proportion of the dry mass of typically cell walls from non-woody plant tissues (about a quarter to half of dry mass may be pectins).

In the method of the invention, the lignocellulosic non-starch carbohydrate comprising material may be in the form of lignocellulose, i.e. a lignocellulosic "substrate" or "biomass" or "feedstock". This encompasses any material containing cellulose, hemicellulose, lignin, protein, and carbohydrates, such as starch and sugar. "Biomass" includes virgin biomass and or non-virgin biomass such as agricultural biomass, commercial organics, construction and demolition debris, municipal solid waste, waste paper and yard waste. Common forms of biomass include trees, shrubs and grasses, wheat, wheat straw, sugar cane bagasse, corn, corn husks, corn kernel including fiber from kernels, products and by-products from milling of grains such as corn (including wet milling and dry milling) as well as municipal solid waste, waste paper and yard waste. "Blended biomass" is any mixture or blend of virgin and non-virgin biomass, preferably having from about 5% to about 95% by weight non-virgin biomass. "Agricultural biomass" includes branches, bushes, canes, corn and corn husks, energy crops, forests, fruits, flowers, grains, grasses, herbaceous crops, leaves, bark, needles, logs, roots, saplings, short rotation woody corps, shrubs, switch grasses, trees, vegetables, vines, and hard and soft woods (not including woods with deleterious materials). In addition, agricultural biomass includes organic waste materials generated from agricultural processes including farming and forestry activities, specifically including forestry wood waste. Agricultural biomass may be any of the aforestated singularly or in any combination of mixture thereof.

Biomass high in starch, sugar, or protein such as corn, grains, fruits and vegetables are usually consumed as food. Conversely, biomass high in cellulose, hemicellulose and lignin are not readily digestible and are primarily utilized for wood and paper products, fuel, or are typically disposed. Generally, the substrate is of high lignocellulose content, including corn stover, rice straw, hay, sugarcane bagasse, and other agricultural biomass, switchgrass, forestry wastes, poplar wood chips, pine wood chips, sawdust, yard waste, and the like, including any combination of substrate.

In a method of the invention, a lignocellulosic non-starch carbohydrate comprising material is contacted with a peroxidase polypeptide as described herein. The lignocellulosic non-starch carbohydrate material may then be contacted with an enzyme which degrades, for example produces sugars from, the said non-starch carbohydrate comprised in the material.

In addition, auxiliary polypeptides can be used in a method of the invention. Such polypeptides may be enzymes. Auxiliary polypeptides may be used at simultaneously with, prior to or subsequent to use of a peroxidase polypeptide described herein.

An auxiliary polypeptide may act so as to further modify the lignocellulosic material comprising a non-starch carbohydrate or may further increase the susceptibility of said lignocellulosic material comprising a non-starch carbohydrate to enzymatic degradation. The auxiliary enzyme may act so as to further increase the effect of a peroxidase polypeptide as described herein.

An auxiliary polypeptide may increase the activity of a non-starch carbohydrate degrading enzyme.

An auxiliary polypeptide may, when contacted with biomass in a reaction, increase the activity of and/or increase the susceptibility to a non-starch carbohydrate degrading enzyme (such as a cellulase).

An auxiliary enzyme can be reacted in the same vessel as a peroxidase polypeptide as described herein and/or a non-starch degrading enzyme (e.g. a cellulase).

While it is understood that many classes of enzymes may function as auxiliary enzymes, in particular auxiliary enzymes can be composed of (but not limited to) enzymes of the following classes: amylases, proteases, lipases, glucuronidases or oxidoreductases.

Polypeptides, for example enzymes, suitable for use in a method of the invention may be composed of enzymes from (1) commercial suppliers; (2) cloned genes expressing enzymes; (3) complex broth (such as that resulting from growth of a microbial strain in media, wherein the strains secrete proteins and enzymes into the media; (4) cell lysates of strains grown as in (3); and, (5) plant material expressing enzymes capable of degrading lignocellulose.

It is recognized that any combination of enzymes (used in addition to the peroxidase described herein) may be utilized. The enzymes may be used alone or in mixtures including, but not limited to, at least a cellulase; at least a hemicellulase; at least a pectinase. That is to say, a method of the invention may be carried out wherein a cellulase is used, for example an endocellulase and/or an exocellulase and/or a β-glucosidase.

It is understood that as described above, an enzyme mix may be composed of a member of each of these enzyme classes, several members of one enzyme class (such as two or more hemicellulases), or any combination of members of these enzyme classes (such as a protease, an exocellulase, and an endoxylanase; or an exoxylanase, and a lipase).

Herein, a cellulase is any polypeptide which is capable of degrading or modifying cellulose. A polypeptide which is capable of degrading cellulose is one which is capable of catalysing the process of breaking down cellulose into smaller units, either partially, for example into cellodextrins, or completely into glucose monomers. A cellulase may give rise to a mixed population of cellodextrins and glucose monomers when contacted with the cellulase. Such degradation will typically take place by way of a hydrolysis reaction.

Herein, a hemicellulase is any polypeptide which is capable of degrading or modifying hemicellulose. That is to say, a hemicellulase may be capable of degrading or modifying one or more of xylan, glucuronoxylan, arabinoxylan, glucomannan and xyloglucan. A polypeptide which is capable of degrading a hemicellulose is one which is capable of catalysing the process of breaking down the hemicellulose into smaller polysaccharides, either partially, for example into oligosaccharides, or completely into sugar monomers, for example hexose or pentose sugar monomers. A hemicellulase may give rise to a mixed population of oligosaccharides and sugar monomers when contacted with the hemicellulase. Such degradation will typically take place by way of a hydrolysis reaction.

Herein, a pectinase is any polypeptide which is capable of degrading or modifying pectin. A polypeptide which is capable of degrading pectin is one which is capable of catalysing the process of breaking down pectin into smaller units, either partially, for example into oligosaccharides, or completely into sugar monomers. A pectinase may give rise to a mixed population of oligosacchardies and sugar monomers when contacted with the pectinase. Such degradation will typically take place by way of a hydrolysis reaction.

"Cellulase" includes both exohydrolases and endohydrolases that are capable of recognizing cellulose, or products resulting from cellulose breakdown, as substrates. Cellulase includes mixtures of enzymes that include endoglucanases, cellobiohydrolases, glucosidases, or any of these enzymes alone, or in combination with other activities. Organisms producing a cellulose-degrading activity often produce a plethora of enzymes with different substrate specificities. Thus, a strain identified as digesting cellulose may be described as having a cellulase, when in fact several enzyme types may contribute to the activity. For example, commercial preparations of 'cellulase' are often mixtures of several enzymes, such as endoglucanase, exoglucanase, and glucosidase activities.

Thus, "cellulase" herein includes mixtures of such enzymes, and includes commercial preparations capable of degrading cellulose, as well as culture supernatant or cell extracts exhibiting cellulose-degrading activity, or acting on the breakdown products of cellulose degradation, such as cellotriose or cellobiose.

"Cellobiohydrolase" or "exoglucanase" or "exocellulase"or "1,4,-β-D-glucan cellobiohydrolase" or "cellulose 1,4-β-cellobiosidase" or "cellobiosidase" includes enzymes that hydrolyze 1,4-β-D-glucosidic linkages in cellulose and cellotetraose, releasing cellobiose from the reducing or non-reducing ends of the chains. Enzymes in group EC 3.2.1.91 include these enzymes such as CBH1 and CBH2.

"β-glucosidase" or "glucosidase" or "β-D-glucoside glucohydrolase" or "cellobiase" EC 3.2.1.21 includes enzymes that release glucose molecules as a product of their catalytic action. These enzymes recognize oligomers of glucose, such as cellobiose (a dimer of glucose linked by β-1,4 bonds) or cellotriose (a trimer of glucose linked by β-1,4 bonds) as substrates. Typically they hydrolyze the terminal, non-reducing β-D-glucose, with release of β-D-glucose.

"Endoglucanase" or "1,4-β-D-glucan 4-glucanohydrolase" or "β-1,4, endocellulase" or "endocellulase", or "cellulase" EC 3.2.1.4 includes enzymes that cleave polymers of glucose attached by β-1,4 linkages. Substrates acted on by these enzymes include cellulose, and modified cellulose substrates such as carboxymethyl cellulose, RBB-cellulose, and the like. EG1, EG2, EG3, EG4, EG5, EG6 and EG7 fall within this class of enzymes.

"Hemicellulase" or "xylanase" includes both exohydrolytic and endohydrolytic enzymes that are capable of recognizing and hydrolyzing hemicellulose, or products resulting from hemicellulose breakdown, as substrates. In monocots, where heteroxylans are the principle constituent of hemicellulose, a combination of endo-1,4-β-xylanase (EC 3.2.1.8) and β-D-xylosidase (EC 3.2.1.37) may be used to break down hemicellulose to xylose. Additional debranching enzymes are capable of hydrolyzing other sugar components (arabinose, galactose, mannose) that are located at branch points in the hemicellulose structure. Additional enzymes are capable of hydrolyzing bonds formed between hemicellulosic sugars (notably arabinose) and lignin. "Endoxylanase" or "1,4-β-endoxylanase" or "1,4-β-D-xylan xylanohydrolase" or (EC 3.2.1.8) include enzymes that hydrolyze xylose polymers attached by β-1,4 linkages. Endoxylanases can be used to hydrolyze the hemicellulose component of lignocellulose as well as purified xylan substrates. "Exoxylanase" or "β-xylosidase" or "xylan 1,4-β-xylosidase" or "1,4-β-D-xylan xylohydrolase" or "xylobiase" or "exo-1,4-β-xylosidase" (EC 3.2.1.37) includes enzymes that hydrolyze successive D-xylose residues from the non-reducing terminus of xylan polymers. "Arabinoxylanase" or "glucuronoarabinoxylan endo-1,4-β-xylanase" or "feraxan endoxylanase" includes enzymes that hydrolyze β-1,4 xylosyl linkages in some xylan substrates.

"Hemicellulase" used in the method of the invention also includes enzymes that hydrolyze the ester linkages (esterase, EC 3.1.1) between xylose units of the xylan polymer and acetyl groups (acetyl xylan esterase, EC 3.1.1.6 and EC 3.1.1.72) or between arabinosyl groups and phenolic moieties such as ferulic acid (feruloyl esterase, EC 3.1.1.73) and p-coumaric acid (coumaroyl esterase, EC 3.1.1-).

Hemicellulase also includes α-L-arabinofuranosidase (EC 3.2.1.55) which remove the arabinose substituent from the xylan backbone. Accordingly, "α-*N*-arabinofuranosidase", "arabinosidase", "α-arabinosidase", "α-L-arabinosidase", "α-arabinofuranosidase", "polysaccharide α-L-arabinofuranosidase", "α-L-arabinofuranoside hydrolase", "L-arabinosidase", "α-L-arabinanase" or "α-L-arabinofuranoside arabinofuranohydrolase" may be used in the invention.

"Hemicellulase" includes enzymes that hydrolyse 1,4-β-D-mannosidic linkages in mannans, galactomannans and/or glucomannans. Accordingly, "mannan endo-1,4-β-mannosidase", "endo-1,4-β-mannanase", "endo-β-1,4-mannase", "β-mannanase B", "β-1, 4-mannan 4-mannanohydrolase", "endo-β-mannanase", "β-D-mannanase" or "1,4-β-D-mannan mannanohydrolase" (EC 3.1.1.78) may be used in the invention.

"Pectinase" used in the method of the invention includes any substance that can hydrolyse a pectin or pectic substance. A composition may comprise any pectinase, for example an endo polygalacturonase, a pectin methyl esterase, an endo-galactanase, a beta galactosidase, a pectin acetyl esterase, an endo-pectin lyase, pectate lyase, alpha rhamnosidase, an exo-galacturonase, an expolygalacturonate lyase, a rhamnogalacturonan hydrolase, a rhamnogalacturonan lyase, a rhamnogalacturonan acetyl esterase, a rhamnogalacturonan galacturonohydrolase, a xylogalacturonase.

Herein, an endo-polygalacturonase (EC 3.2.1.15) is any polypeptide which is capable of catalyzing the random hydrolysis of 1,4-α-D-galactosiduronic linkages in pectate and other galacturonans. This enzyme may also be referred to as polygalacturonase pectin depolymerase, pectinase, endopolygalacturonase, pectolase, pectin hydrolase, pectin polygalacturonase, poly-α-1,4-galacturonide glycanohydrolase, endogalacturonase; endo-D-galacturonase or poly(1,4-α-D-galacturonide) glycanohydrolase.

Herein, a pectin methyl esterase (EC 3.1.1.11) is any enzyme which is capable of catalyzing the reaction: pectin + n H₂O = n methanol + pectate. The enzyme may also been known as pectinesterase, pectin demethoxylase, pectin methoxylase, pectin methylesterase, pectase, pectinoesterase or pectin pectylhydrolase.

Herein, an endo-galactanase (EC 3.2.1.89, EC 3.2.1.90) is any enzyme capable of catalyzing the endohydrolysis of 1,4-β-D-galactosidic linkages in arabinogalactans. The enzyme may also be known as arabinogalactan endo-1,4-β-galactosidase, endo-1,4-β-galactanase, galactanase, arabinogalactanase or arabinogalactan 4-β-D-galactanohydrolase.

Herein, a pectin acetyl esterase is defined herein as any enzyme which has an acetyl esterase activity which catalyzes the deacetylation of the acetyl groups at the hydroxyl groups of GalUA residues of pectin

Herein, an endo-pectin lyase (EC 4.2.2.10) is any enzyme capable of catalyzing the eliminative cleavage of (1 →4)-α-D-galacturonan methyl ester to give oligosaccharides with 4-deoxy-6-*O*-methyl-α-D-galact-4-enuronosyl groups at their non-reducing ends. The enzyme may also be known as pectin lyase, pectin *trans*-eliminase; endo-pectin lyase, polymethylgalacturonic transeliminase, pectin methyltranseliminase, pectolyase, PL, PNL or PMGL or (1 →4)-6-*O*-methyl-α-D-galacturonan lyase.

Herein, a pectate lyase (EC 4.2.2.2) is any enzyme capable of catalyzing the eliminative cleavage of (1 →4)-α-D-galacturonan to give oligosaccharides with 4-deoxy-α-D-galact-4-enuronosyl groups at their non-reducing ends. The enzyme may also be known polygalacturonic transeliminase, pectic acid transeliminase, polygalacturonate lyase, endopectin methyltranseliminase, pectate transeliminase, endogalacturonate transeliminase, pectic acid lyase, pectic lyase, α-1,4-D-endopolygalacturonic acid lyase, PGA lyase, PPase-N, endo-α-1,4-polygalacturonic acid lyase, polygalacturonic acid lyase, pectin *trans*-eliminase, polygalacturonic acid *trans*-eliminase or (1→4)-α-D-galacturonan lyase.

Herein, an alpha rhamnosidase (EC 3.2.1.40) is any polypeptide which is capable of catalyzing the hydrolysis of terminal non-reducing α-L-rhamnose residues in α-L-rhamnosides or alternatively in rhamnogalacturonan. This enzyme may also be known as α-L-rhamnosidase T, α-L-rhamnosidase N or α-L-rhamnoside rhamnohydrolase.

Herein, exo-galacturonase (EC 3.2.1.82) is any polypeptide capable of hydrolysis of pectic acid from the non-reducing end, releasing digalacturonate. The enzyme may also be known as exo-poly-α-galacturonosidase, exopolygalacturonosidase or exopolygalacturanosidase.

Herein, exo-galacturonase (EC 3.2.1.67) is any polypeptide capable of catalyzing: (1,4-α-D-galacturonide)*ₙ* + H₂O = (1,4-α-D-galacturonide)_{*n*-1} + D-galacturonate. The enzyme may also be known as galacturan 1,4-α-galacturonidase, exopolygalacturonase, poly(galacturonate) hydrolase, exo-D-galacturonase, exo-D-galacturonanase, exopoly-D-galacturonase or poly(1,4-α-D-galacturonide) galacturonohydrolase.

Herein, exopolygalacturonate lyase (EC 4.2.2.9) is any polypeptide capable of catalyzing eliminative cleavage of 4-(4-deoxy-α-D-galact-4-enuronosyly)-D-galacturonate from the reducing end of pectate, i.e. de-esterified pectin. This enzyme may be known as pectate disaccharide-lyase, pectate exo-lyase, exopectic acid transeliminase, exopectate lyase, exopolygalacturonic acid-*trans*-eliminase, PATE, exo-PATE, exo-PGL or (1 →)-α-D-galacturonan reducing-end-disaccharide-lyase.

Herein, rhamnogalacturonan hydrolase is any polypeptide which is capable of hydrolyzing the linkage between galactosyluronic acid acid and rhamnopyranosyl in an endo-fashion in strictly alternating rhamnogalacturonan structures, consisting of the disaccharide [(1,2-alpha-L-rhamnoyl-(1,4)-alpha-galactosyluronic acid].

Herein, rhamnogalacturonan lyase is any polypeptide which is any polypeptide which is capable of cleaving α-L-Rha*p*-(1 →4)-α-D-Gal*p*A linkages in an endo-fashion in rhamnogalacturonan by beta-elimination.

Herein, rhamnogalacturonan acetyl esterase is any polypeptide which catalyzes the deacetylation of the backbone of alternating rhamnose and galacturonic acid residues in rhamnogalacturonan.

Herein, rhamnogalacturonan galacturonohydrolase is any polypeptide which is capable of hydrolyzing galacturonic acid from the non-reducing end of strictly alternating rhamnogalacturonan structures in an exo-fashion.

Herein, xylogalacturonase is any polypeptide which acts on xylogalacturonan by cleaving the β-xylose substituted galacturonic acid backbone in an *endo*-manner. This enzyme may also be known as xylogalacturonan hydrolase.

Herein, an α-L-arabinofuranosidase (EC 3.2.1.55) is any polypeptide which is capable of acting on α-L-arabinofuranosides, α-L-arabinans containing (1,2) and/or (1,3)-and/or (1,5)-linkages, arabinoxylans and arabinogalactans. This enzyme may also be referred to as α-N-arabinofuranosidase, arabinofuranosidase or arabinosidase.

Herein, endo-arabinanase (EC 3.2.1.99) is any polypeptide which is capable of catalyzing endohydrolysis of 1,5-α-arabinofuranosidic linkages in 1,5-arabinans. The enzyme may also be know as endo-arabinase, arabinan endo-1,5-α-L-arabinosidase, endo-1,5-α-L-arabinanase, endo-α-1,5-arabanase; endo-arabanase or 1,5-α-L-arabinan 1,5-α-L-arabinanohydrolase.

"Amylase" or "alpha glucosidase" includes enzymes that hydrolyze 1,4-[alpha]-glucosidic linkages in oligosaccharides and polysaccharides.

"Protease" includes enzymes that hydrolyze peptide bonds (peptidases), as well as enzymes that hydrolyze bonds between peptides and other moieties, such as sugars (glycopeptidases). Many proteases are characterized under EC 3.4, and are suitable for use in the invention. Some specific types of proteases include, cysteine proteases including pepsin, papain and serine proteases including chymotrypsins, carboxypeptidases and metalloendopeptidases.

"Lipase" includes enzymes that hydrolyze lipids, fatty acids, and acylglycerides, including phospoglycerides, lipoproteins, diacylglycerols, and the like. In plants, lipids are used as structural components to limit water loss and pathogen infection. These lipids include waxes derived from fatty acids, as well as cutin and suberin.

"Glucuronidase" includes enzymes that catalyze the hydrolysis of β-glucuronoside to yield an alcohol and glucuronate. Many glucuronidases have been characterized and may be suitable for use in the invention, for example β-glucuronidase (EC 3.2.1.31), hyalurono-glucuronidase (EC 3.2.1.36), glucuronosyl-disulfoglucosamine glucuronidase (3.2.1.56), glycyrrhizinate β-glucuronidase (3.2.1.128) or α-D-glucuronidase (EC 3.2.1.139).

"Oxidoreductase" is any enzyme that catalyzes the transfer of electrons from one molecule (the reductant, also called the hydrogen or electron donor) to another (the oxidant, also called the hydrogen or electron acceptor), for example an enzyme falling within EC 1. An example of such an enzyme which may be used in a method of the invention is glucose oxidase (EC 1.1.3.4), i.e. an enzyme which catalyzes the oxidation of beta-D-glucose into D-glucono-1,5-lactone.

In such a method described above, a combination of enzymes may be used, either simultaneously, separately or sequentially, to acts on, for example, a lignocellulosic substrate or plant biomass, serving as the feedstock, so as to convert this complex substrate to simple sugars and oligosaccharides for the production of ethanol or other useful product.

Accordingly, another aspect of the invention includes methods that utilize mixtures of the enzymes as described above, either simultaneously, separately or sequentially, optionally together with further enzymes or physical treatments such as temperature and pH to convert the lignocellulosic plant biomass to sugars and oligosaccharides.

Enzyme combinations or physical treatments can be administered concomitantly, or sequentially or separately. The enzymes can be produced either exogenously in microorganisms, yeasts, fungi, bacteria or plants, then isolated and added to the lignocellulosic feedstock. Alternatively, the enzymes are produced, but not isolated, and crude cell mass fermentation broth, or plant material (such as corn stover), and the like are added to the feedstock. Alternatively, the crude cell mass or enzyme production medium or plant material may be treated to prevent further microbial growth (for example, by heating or addition of antimicrobial agents), then added to the feedstock. These crude enzyme mixtures may include the organism producing the enzyme. Alternatively, the enzyme may be produced in a fermentation that uses feedstock (such as corn stover) to provide nutrition to an organism that produces an enzyme(s). In this manner, plants that produce the enzymes may serve as the lignocellulosic feedstock and be added into lignocellulosic feedstock.

While the auxiliary enzymes have been discussed as a mixture it is recognized that the enzymes may be added sequentially where the temperature, pH, and other conditions may be altered to increase the activity of each individual enzyme. Alternatively, an optimum pH and temperature can be determined for the enzyme mixture.

A polypeptide may be used prior to, at the same time as, or subsequent to any pre-treatment step(s). A polypeptide may, alternatively, or in addition, be used in combination with a non-starch carbohydrate degrading enzyme (such as a cellulase). Accordingly, a polypeptide as described herein may be used as part of a pre-treatment protocol, thus reducing chemical and/or energy input as compared with a conventional pre-treatment protocol. A polypeptide as described herein invention may be used a part of a saccharification protocol. Use of the polypeptide may allow less non-starch degrading enzyme to be used or may allow a process requiring less time to be carried out.

A method of the invention may be carried out, i.e. reacted with non-starch carbohydrate comprising substrate, under mild conditions that do not include extreme heat or acid treatment, as is currently utilized for biomass conversion using bioreactors. A peroxidase polypeptide as described herein may be used simultaneously, separately or sequentially with one or more of the other polypeptides or enzymes herein described.

For example, the enzymes can be incubated at about 25°C, about 30 °C, about 35 °C, about 37 °C, about 40 °C, about 45 °C, about 50 °C, or about 55 °C. That is, they can be incubated at from about 20 °C to about 70 °C , in buffers of low to medium ionic strength, and neutral pH. By "medium ionic strength" is intended that the buffer has an ion concentration of about 200 millimolar (mM) or less for any single ion component. The pH may range from about pH 2.5, about pH 3.0, about pH 3.5, about pH 4.0, about pH 4.5, about pH 5, about pH 5.5, about pH 6, about pH 6.5, about pH 7, about pH 7.5, about pH 8.0, to about pH 8.5. Generally, the pH range will be from about pH 3.0 to about pH 9.

Incubation of enzyme combinations described herein, i.e. a peroxidase polypeptide and non-starch carbohydrate degrading enzyme, for example under the conditions set out above, may result in release or liberation of sugar from the substrate, for example lignocellulose. The amount of sugar liberated may be at least about 20%, at least about 30%, at least about 40%, at least about 50%, at least about 60%, at least about 70%, at least about 80%, at least about 90%, at least about 95% or more of available sugar.

The treatment with a non-carbohydrate degrading enzyme may occur from several minutes to several hours, such as from about 6 hours to about 120 hours, preferably about 12 hours to about 72 hours, more preferably about 24 to 48 hours.

A pretreatment step involving incubation with an enzyme or enzyme mixture can be utilized. The pretreatment step can be performed at many different temperatures but it is preferred that the pretreatment occur at the temperature best suited to the enzyme mix being tested, or the predicted enzyme optimum of the enzymes to be tested. The temperature of the pretreatment may range from about 10 °C to about 80 °C, about 20 °C to about 80 °C, about 30 °C to about 70 °C, about 40 °C to about 60 °C, about 37 °C to about 50 °C, preferably about 37 °C to about 80 °C, more preferably about 50 °C. In the absence of data on the temperature optimum, it is preferable to perform the pretreatment reactions at 37 °C first, then at a higher temperature such as 50 °C. The pH of the pretreatment mixture may range from about 2.0 to about 10.0, but is preferably about 3.0 to about 7.0, more preferably about 4.0 to about 6.0, even more preferably about 4.5 to about 5. Again, the pH may be adjusted to maximize enzyme activity and may be adjusted with the addition of the enzyme. Comparison of the results of the assay results from this test will allow one to modify the method to best suit the enzymes being tested.

The pretreatment reaction may occur from several minutes to several hours, such as from about 6 hours to about 120 hours, preferably about 6 hours to about 48 hours, more preferably about 6 to about 24 hours, most preferably for about 6 hours.

If a peroxidase polypeptide is used in pre-treatment, this may allow the mild conditions described above to be used and to avoid the use of extreme heat or acid treatment, as is currently utilized for biomass conversion using bioreactors.

A method of the invention for producing a sugar or sugars is typically a process for converting a complex carbohydrate such as lignocellulose into sugars, preferably fermentable sugars. Such a process may be referred to as "saccharification." Accordingly, a method of the invention may result in the liberation of one or more hexose and/or pentose sugars, such as one or more of glucose, xylose, arabinose, galactose, D-galacturonic acid, mannose, rhamnose, sucrose and fructose.

The fermentable sugars can be converted to useful value-added fermentation products, non-limiting examples of which include amino acids, vitamins, pharmaceuticals, animal feed supplements, specialty chemicals, chemical feedstocks, plastics, solvents, fuels, or other organic polymers, lactic acid, and ethanol, including fuel ethanol.

Specific value-added products that may be produced by the methods of the invention include, but not limited to, biofuels (including ethanol and butanol and a biogas); lactic acid; a plastic; a specialty chemical; an organic acid, including citric acid, succinic acid, fumaric acid, itaconic acid and maleic acid; 3-hydoxy-propionic acid, acrylic acid; acetic acid; 1,3-propane-diol; ethylene, glycerol; a solvent; an animal feed supplement; a pharmaceutical, such as a β-lactam antibiotic or a cephalosporin; vitamins; an amino acid, such as lysine, methionine, tryptophan, threonine, and aspartic acid; an industrial enzyme, such as a protease, a cellulase, an amylase, a glucanase, a lactase, a lipase, a lyase, an oxidoreductases, a transferase or a xylanase; and a chemical feedstock.

Accordingly, the disclosure provides a method for the preparation of a fermentation product, which method comprises:
a. degrading a non-starch carbohydrate, for example producing a sugar or sugars from a non-starch carbohydrate, using a method as described herein; and
b. fermenting the resulting material,
   thereby to prepare a fermentation product.

The fermentation product may be an amino acid, a vitamin, a pharmaceutical, an animal feed supplement, a specialty chemical, a chemical feedstock, a plastic, ethanol, including fuel ethanol the term "ethanol" being understood to include ethyl alcohol or mixtures of ethyl alcohol and water). More specific fermentation products include lactic acid, 3-hydroxy-propionic acid, acrylic acid, acetic acid, succinic acid, citric acid, an amino acid, 1,3-propane-diol, ethylene, glycerol, a β-lactam antibiotic or a cephalosporin.

A method for the preparation of a fermentation product may optionally comprise recovery of the fermentation product.

Such a process may be carried out under aerobic or anaerobic conditions. Preferably, the process is carried out under micro-aerophilic or oxygen limited conditions.

An anaerobic fermentation process is herein defined as a fermentation process run in the absence of oxygen or in which substantially no oxygen is consumed, preferably less than 5, 2.5 or 1 mmol/L/h, and wherein organic molecules serve as both electron donor and electron acceptors.

An oxygen-limited fermentation process is a process in which the oxygen consumption is limited by the oxygen transfer from the gas to the liquid. The degree of oxygen limitation is determined by the amount and composition of the ingoing gasflow as well as the actual mixing/mass transfer properties of the fermentation equipment used. Preferably, in a process under oxygen-limited conditions, the rate of oxygen consumption is at least 5.5, more preferably at least 6 and even more preferably at least 7 mmol/L/h.

The disclosure also provides use of a polypeptide having peroxidase activity as described herein for use in a method for degrading lignin. In such use the lignin may be in the form of lignocellulose.

The following Examples illustrate the invention:

### Example 1

### Lignin model compound degradation by peroxidases obtained from Marasmius scorodonius

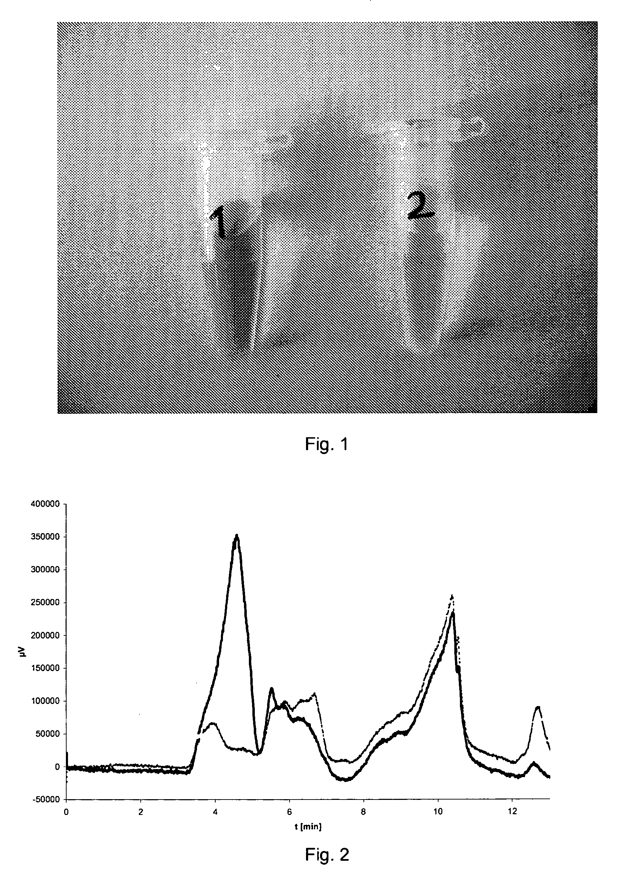

2-Methoxyphenol (guaiacol), veratryl alcohol, ferulic acid, and coumaric acid served as low molecular weight lignin model compounds. 2 mM of each substrate were incubated with 1.5 µL of enzyme solution and 15 µL of H₂0₂ (20 mM) in sodium acetate buffer (50 mM, 1.5 mL total sample volume, pH 6.0, 27 °C). After 60 min, another 15 µL of H₂0₂ were added, and the reaction was stopped after 2 h. A brownish color was observed with guaiacol (see Figure 1) and ferulic acid. No visually observable changes occurred with the coumaric acid and the veratryl alcohol samples.

Lignin organosolv^{™} (brown powder, Sigma-Aldrich) was used to evaluate the effect of MsP1 on an insoluble high molecular weight lignin. 2.5 mg of lignin organosolv were mixed with 20 µL of MsP1 solution (either active or heat inactivated) in sodium acetate buffer (50 mM, pH 3 - 6, total sample volume 1.5 mL) and 100 µL of hydrogen peroxide solution (20 mM). Another 100 µL of hydrogen peroxide solution were added after 60 min. The total reaction time was 120 min (incubation at 27 °C on a rotary shaker). The solid was removed by centrifugation, and the supernatant was subjected to HPLC analysis. The columns used were from Polymer laboratoriesTM PL aquagel-OH MIXED and PL aquagel-OH 20, the UV detection was performed at 195 nm. As the lignin material is insoluble in water, the supernatant of the samples with heat inactivated enzymes remained colourless. When the active enzyme was used, a red to brownish colour was observed in the supernatants. High molecular weight compounds released by the enzyme treatment were traceable by means of size exclusion chromatography (see Figure 2).

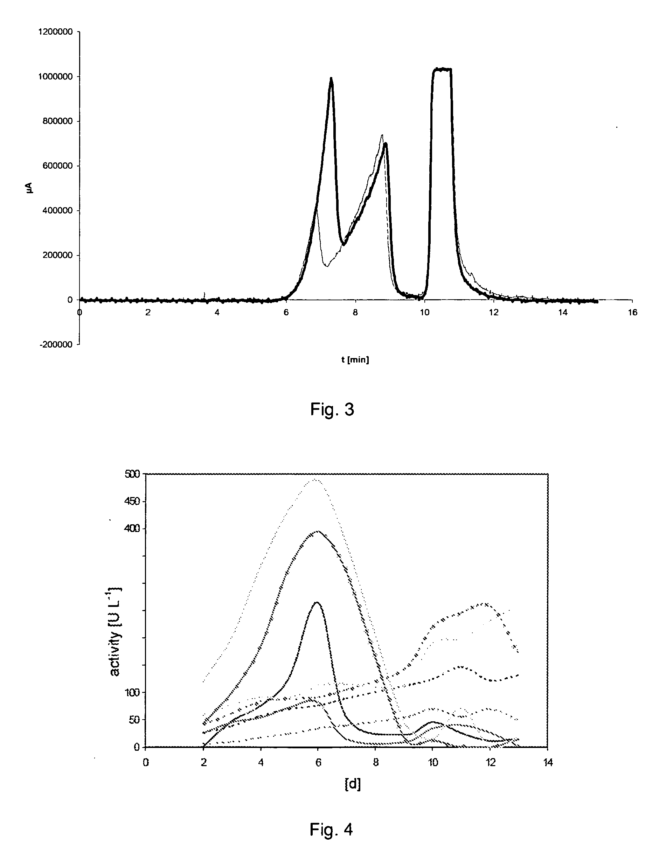

To produce H₂0₂ continuously, a glucose/glucose oxidase system was applied. 500 mg of lignin (organosolv), 4 mL MsP1, 20 mmoL glucose, and 10 µL (10 U) glucose oxidase were incubated in 216 mL sodium acetate buffer (50 mM, pH 6.0) for 24 h and 140 rpm at room temperature. The supernatants were separated by means of centrifugation and analyzed by HPLC (DAD and evaporative light scattering (ELSD) detection). A significant solubilisation of formerly insoluble material was detected (see Figure 3).

After running the assay, the solids were centrifuged and dried. When heat-inactivated peroxidase was used, a loss of pellet dry weight of 2.5% was observed. With active peroxidase, the loss was 7.3%. Thus, the lignin is partly solved as a result of the enzyme's activity.

### Example 2 (not according to the invention)

### Chemical analysis of corn stover

The chemical composition of corn stover was characterized by determination of the Kjeldahl nitrogen (protein), Soxhlet extraction (lipids), orcinol-sulfuric acid assay (carbohydrates), and by the acetyl bromide method (lignin) (Table 1).

**Table 1: Composition of corn stover**

| Parameter | Corn stover |
|---|---|
| Dry mass [%] | 98.7 |
| Protein [% in DM] | 12.7 |
| Carbohydrates [% in DM] | 67.2 |
| Lipids [% in DM] | 0.5 |
| Lignin [% in DM] | 20.4 |
| ∑ | 100.8 |

### Example 3 (not according to the invention)

### Secretome of Marasmius scorodonius

To characterize the extra-cellular enzymes involved in the natural process of lignin degradation, *M. scorodonius* was grown in submerged cultures using corn stover as a carbon and nitrogen source. Pre-cultures were grown in 100 mL of standard solution (SNS) and four different media were used for the main culture:

| | | |
|---|---|---|
| • Medium A: | glucose | (1.00%) |
| | MgSO₄ | (0.05%) |
| | KH₂PO₄ | (0.15%) |
| | yeast extract | (0.20%) |
| | trace element solution | 1.0mlL⁻¹ |
| • Medium B: | like medium A, but without glucose | |
| • Medium C: | like medium A, but without yeast extract | |
| • Medium D: | like medium A, but with 0.4% of Tween80 instead of glucose | |

The main cultures were inoculated with 20 mL of the pre-cultures. Samples were drawn from the culture supernatants (depending on the growth rate of the fungal mycelium), and the secretome was analyzed by means of enzyme assays and by electrophoretic techniques.

### Laccases and peroxidases

Laccase and peroxidase activities were quantified by the ABTS assay, with and without addition of H₂0₂ respectively (see Figure 4).

Highest peroxidase activities were observed in the cultures grown without glucose (medium B and D).

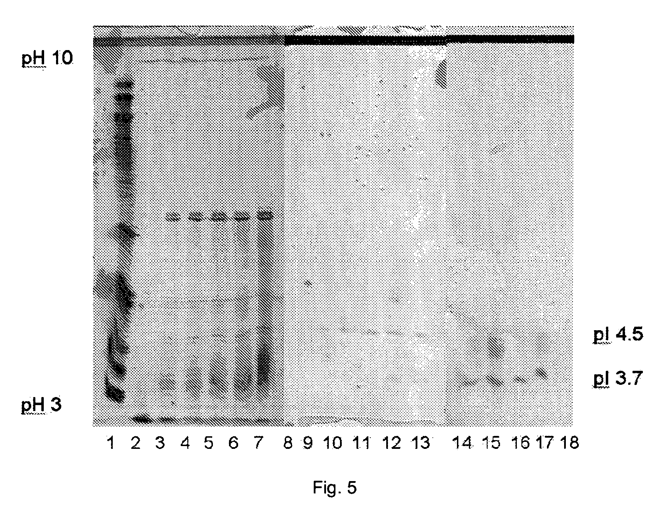

Activity stained IEF-gels indicated the presence of laccase (pl 4.5) and peroxidase (pl 3.7) type enzymes (see Figure 5). As no activity bands were observed in the cultures grown in SNS, these enzymes are inducible by the renewable material 'corn stover'.

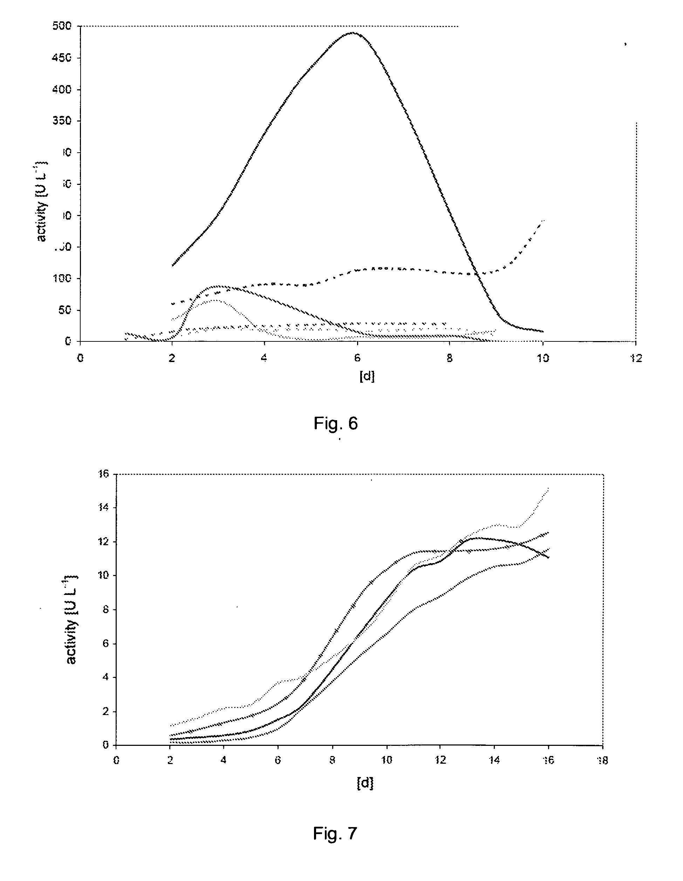

Compared to cultures grown in SNS supplemented with lignin (0.1 %), high peroxidase activities were detected in the cultures containing corn stover in medium D (see Figure 6).

### Peptidases

Only very low peptidase activities were detected in the cultures supplemented with corn stover.

### β-Glucosidases

4-Nitrophenol-β-D-glucoside served as a substrate to quantify β-glucosidase activity in the submerged cultures of *M. scorcdonius.* After hydrolysis, the concentration of 4-nitrophenol was quantified by means of UV-Vis spectrophotometry at λ=400 nm (see Figure 7).

On culture day 16, the activities were still increasing in media B, C, and D.

### Esterases

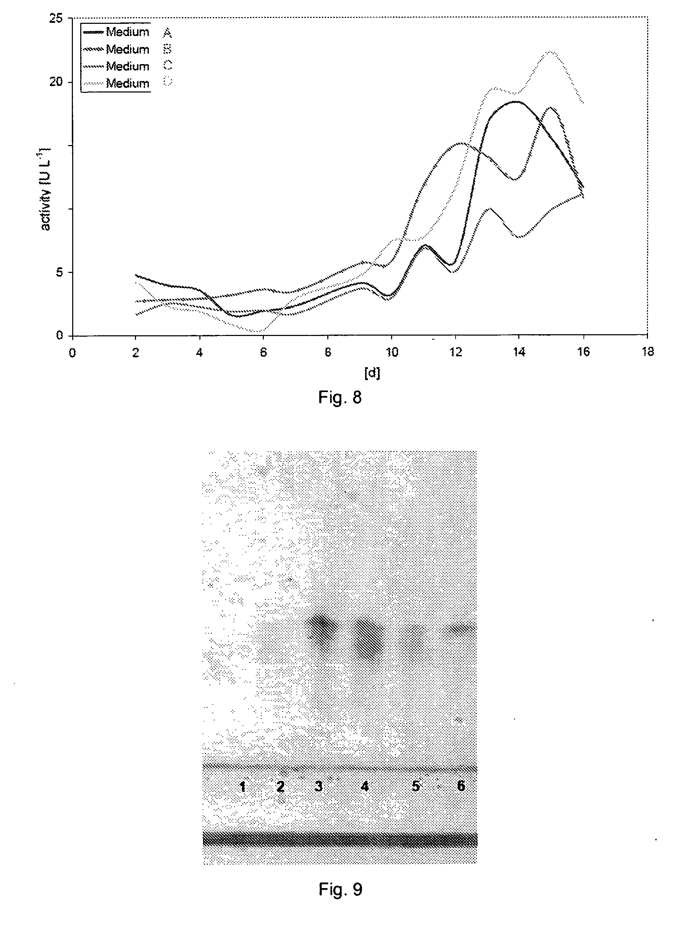

Esterolytic activity was quantified by using nitrophenol butyrate as a substrate (see Figure 8).

By activity staining using fast Blue SB and 1-naphthyl acetate, the esterolytic activity was visualized directly on an IEF gel (see Figure 9).

### Example 4

### Release of fermentable sugars from corn stover

The corn stover substrate used in the hydrolysis experiments was pre-treated according to the following protocol:
- milling (∅ < 1 mm),;
- soaking (5 g corn stover + 117 mL water, l h);
- heating (microwave, 700W, 3 mm);
- washing (800 mL water)
- drying

Subsequently, 50mg of the pre-treated corn stover were dispersed in 1.5 mL of sodium acetate buffer (50 mM, pH 3.5), and 6 U of MsP1, and 7.5 U of cellulase *(Trichoderma reesei,* Sigma C8546) was added. The cellulase activity was determined using a 1% 2-hydroxyethyl cellulose solution in 0.5M sodium acetate buffer.

In one corn stover mixture H₂0₂ (total amount of 3.2 µmol) was added periodically, in another mixture no H₂0₂ was added. Both samples were incubated at 30°C and in time samples were taken for analysis of the glucose-monomer content. Glucose monomer content was determined using the reducing sugar assay of Nelson Somogyi.

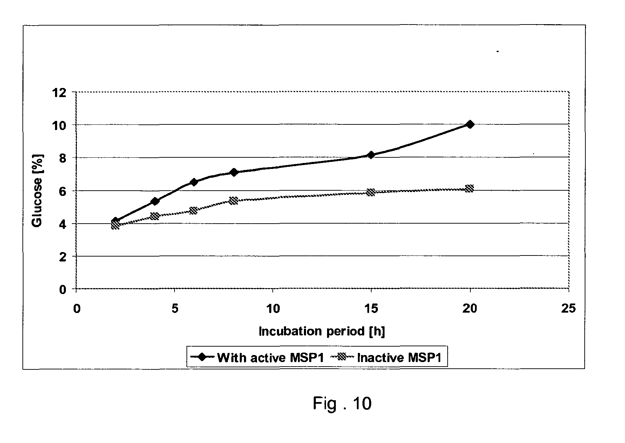

In figure 10, the release of glucose monomer as percentage of the corn stover dry matter is presented, for both active MsP1 (including H₂0₂ addition) and inactive MsP1 (without H₂0₂ addition). It is clearly shown that active MsP1 in the incubation mixture results in additional glucose to be released from the corn stover by the Trichoderma cellulase.

## Claims

1. A method for the oxidation of lignocellulosic material, which method comprises contacting said lignocellulosic material with a polypeptide which has peroxidase activity and which is:
a. a polypeptide comprising an amino acid sequence having at least 55% homology with an amino acid sequence set out In any one of: amino acids 21 to 513 of SEQ NO: 2; amino acids 20 to 510 of SEQ ID NO: 4; amino acids 1 to 493 of SEQ ID NO 6; or amino acids 1 to 491 of SEQ ID NO: 8 or
b. a polypeptide encoded by a polynucleotide comprising a nucleotide sequence having at least 55% homology with the nucleotide sequence set out in any one of: nucleotides 61 to 1539 of SEQ ID NO: 1; nucleotides 58 to 1530 of SEQ ID NO: 3; nucleotides 1 to 1479 of SEQ ID NO: 5; or nucleotides 1 to 1473 of SEQ ID NO: 7.

2. A method according to claim 1, wherein the oxidation is increasing the susceptibility of the lignocellulosic material to enzymatic degradation.

3. A method for the production of a sugar or sugars from a lignocellulosic material, which method comprises the following steps:
(i) pre-treatment of the lignocellulosic material;
(ii) oxidation of said lignocellulosic material using a method according to claim 1 or 2; and
(ii) contacting the thus-modified lignocellulosic material with one or more cellulase and/or one or more hemicellulase and/or one or more pectinase.

4. A method according to claim 3, wherein the polypeptide which has peroxidase activity is contacted with the material comprising a non-starch carbohydrate during, prior to, or subsequent to, the pre-treatment step.

5. A method according to any one of the preceding claims, wherein the polypeptide which has peroxidase activity is contacted with the lignocellulosic material in combination with an auxiliary enzyme, which auxiliary enzyme further modifies said material comprising a non-starch carbohydrate or increases the susceptibility of said material comprising a non-starch carbohydrate to enzymatic degradation, wherein the auxiliary enzyme is an enzyme of the classes: amylases, proteases, lipases, glucuronidases or oxidoreductases.

6. A method according to any one of the preceding claims, wherein the polypeptide having peroxidase activity is encoded by a polynucleotide obtainable from *Marasmius scorodonlus.*

7. A method according to any of the preceding claims, wherein the lignocellulosic material is orchard primings, chaparral, mill waste, urban wood waste, municipal waste, logging waste, forest thinnings, short- rotation woody crops, industrial waste, wheat straw, oat straw, rice straw, barley straw, rye straw, flax straw, soy hulls, rice hulls, rice straw, corn gluten feed, oat hulls, sugar cane, corn stover, corn stalks, corn cobs, corn husks, prairie grass, gamagrass, foxtail, sugar beet pulp, citrus fruit pulp, seed hulls, cellulosic animal wastes, lawn clippings, cotton, seaweed, trees, shrubs, grasses, wheat, wheat straw, sugar cane bagasse, corn, corn husks, corn hobs, corn Kernel, fiber from kernels, products and by-products from wet or dry milling of grains, municipal solid waste, waste paper, yard waste, herbaceous material, agricultural residues, forestry residues, municipal solid waste, waste paper, pulp, paper mill residues, branches, bushes, canes, corn, corn husks, an energy crop, forest, a fruit, a flower, a grain, a grass, a herbaceous crop, a leaf, bark, a needle, a log, a root, a sapling, a shrub, switch grass, a tree, a vegetable, fruit peel, a vine, sugar beet pulp, wheat midlings, oat hulls, hard or soft wood, organic waste material generated from an agricultural process or forestry wood waste, or a combination of any two or more thereof.

8. A method for the preparation of a fermentation product, which method comprises:
a treating a lignocellulosic material according to a method of any one of claims 1-7 and
b. fermenting the resulting material,
thereby to prepare a fermentation product.

9. A method according to claim 8, wherein the fermentation product is ethanol, butanol; lactic acid, 3-hydroxy-propionic acid, acrylic acid, acetic acid, succinic acid, citric acid, malic acid, fumaric acid, itaconic acid, an amino add, 1,3-propane-diol, ethylene, glycerol, a β-lactam antibiotic or a cephalosporin.

## Patentansprüche

1. Verfahren zur Oxidation von lignocellulosehaltigem Material, wobei das Verfahren umfasst, dass man das lignocellulosehaltige Material mit einem Polypeptid, das Peroxidaseaktivität aufweist und das Folgendes ist:
a. ein Polypeptid mit einer Aminosäuresequenz mit mindestens 55% Homologie zu einer Aminosäuresequenz gemäß einer der folgenden: Aminosäuren 21 bis 513 von SEQ NO: 2; Aminosäuren 20 bis 510 von SEQ ID NO: 4; Aminosäuren 1 bis 493 von SEQ ID NO: 6; oder Aminosäuren 1 bis 491 von SEQ ID NO: 8; oder
b. ein Polypeptid, das von einem Polynukleotid codiert wird, das eine Nukleotidsequenz mit mindestens 55% Homologie zu der Nukleotidsequenz in einem der folgenden aufweist: Nukleotide 61 bis 1539 von SEQ ID NO: 1, Nukleotide 58 bis 1530 von SEQ ID NO: 3; Nukleotide 1 bis 1479 von SEQ ID NO: 5; oder Nukleotide 1 bis 1473 von SEQ ID NO: 7 in Kontakt bringt.

2. Verfahren nach Anspruch 1, wobei die Oxidation die Zugänglichkeit des lignocellulosehaltigen Materials für einen enzymatischen Abbau erhöht.

3. Verfahren zur Herstellung eines Zuckers bzw. von Zuckern aus einem lignocellulosehaltigen Material, wobei das Verfahren die folgenden Schritte umfasst:
(i) Vorbehandlung des lignocellulosehaltigen Materials;
(ii) Oxidation des lignocellulosehaltigen Materials mit einem Verfahren nach Anspruch 1 oder 2; und
(iii) Inkontaktbringen des so modifizierten lignocellulosehaltigen Materials mit einer oder mehreren Cellulase(n) und/oder einer oder mehreren Hemicellulase(n) und/oder einer oder mehreren Pectinase (n) .

4. Verfahren nach Anspruch 3, wobei man das Polypeptid mit Peroxidaseaktivität mit dem Material, das ein Nichtstärkekohlehydrat umfasst, während des Vorbehandlungsschritts oder vor oder nach dem Vorbehandlungsschritt in Kontakt bringt.

5. Verfahren nach einem der vorhergehenden Ansprüche, wobei das Polypeptid mit Peroxidaseaktivität mit dem lignocellulosehaltigen Material in Kombination mit einem Hilfsenzym in Kontakt gebracht wird, wobei das Hilfsenzym das Material, das ein Nichtstärkekohlehydrat umfasst, weiter modifiziert oder die Zugänglichkeit des Materials, das ein Nichtstärkekohlehydrat umfasst, für enzymatischen Abbau erhöht, wobei es sich bei dem Hilfsenzym um ein Enzym der Klassen Amylasen, Proteasen, Lipasen, Glukuronidasen oder Oxidoreduktasen handelt.

6. Verfahren nach einem der vorhergehenden Ansprüche, wobei das Polypeptid mit Peroxidaseaktivität von einem Polynukleotid, das von *Marasmius scorodonlus* erhältlich ist, codiert wird.

7. Verfahren nach einem der vorhergehenden Ansprüche, wobei es sich bei dem lignocellulosehaltigen Material um Folgendes handelt: Obstbaumschnittmaterial, Chaparral, Fabrikabfall, Stadtwaldabfall, städtischen Abfall, beim Holzeinschlag entstehenden Abfall, Schwachholz aus der Forstpflege, Kurzumtriebsholz, Industrieabfall, Weizenstroh, Haferstroh, Reisstroh, Gerstenstroh, Roggenstroh, Flachsstroh, Sojabohnenschalen, Reisspelzen, Maisglutenfutter, Haferspelzen, Zuckerrohr, Mais-Stover, Maisstängel, Maiskolben, Maislieschen, Präriegras, gamma-Gras, Fuchsschwanzgras, ausgelaugte Zuckerrübenschnitzel, Zitrusfruchtfleisch, Samenschalen, cellulosehaltige tierische Abfälle, Rasenschnitt, Baumwolle, Meeresalgen, Bäume, Sträucher, Gräser, Weizen, Zuckerrübenbagasse, Mais, Maiskorn, Fasern aus Körnern, Produkte und Nebenprodukte der Trocken- oder Nassvermahlung von Getreide, festen städtischer Abfall, Abfallpapier, Hof- und Gartenabfälle, krautiges Material, Rückstände der Land - und Forstwirtschaft, Zellstoff, Abfälle der Papierindustrie, Zweige, Büsche, Rohre, eine Energiepflanzenkultur, Forst, eine Frucht, eine Blüte, ein Korn, eine krautige Kulturpflanze, ein Blatt, Rinde, eine Nadel, einen Baumstamm, eine Wurzel, eine Jungpflanze, einen Strauch, Rutenhirse, ein Gemüse, Fruchtschalen, eine Rankpflanze, Weizenkleie, Hart - oder Weichholz, organisches Abfallmaterial, das durch einen landwirtschaftlichen Vorgang entsteht, oder Abfallmaterial von Forsten, oder eine Kombination von zwei oder mehr davon.

8. Verfahren zur Herstellung eines Fermentationsprodukts, das Folgendes umfasst:
a. Behandeln eines lignocellulosehaltigen Materials gemäß einem Verfahren nach einem der Ansprüche 1-7 und
b. Fermentieren des erhaltenen Materials, wodurch man zu einem Fermentationsprodukt gelangt.

9. Verfahren nach Anspruch 8, wobei es sich bei dem Fermentationsprodukt um Ethanol, Butanol, Milchsäure, 3-Hydroxypropionsäure, Acrylsäure, Essigsäure, Bernsteinsäure, Citronensäure, Äpfelsäure, Fumarsäure, Itaconsäure, eine Aminosäure, 1,3-Propandiol, Ethylen, Glycerol, ein β-Lactam-Antibiotikum oder ein Cephalosporin handelt.

## Revendications

1. Procédé d'oxydation d'un matériel ligno-cellulosique, lequel procédé comprend la mise en contact dudit matériel ligno-cellulosique avec un polypeptide qui a une activité de peroxydase et qui est :
a. un polypeptide comprenant une séquence d'acides aminés ayant une homologie d'au moins 55% avec une séquence d'acides aminés indiquée dans l'un quelconque des : acides aminés 21 à 513 de la SEQ ID n° : 2 ; acides aminés 20 à 510 de la SEQ ID n° : 4 ; acides aminés 1 à 493 de la SEQ ID n° : 6 ; ou acides aminés 1 à 491 de la SEQ ID n° : 8 ; ou
b. un polypeptide codé par un polynucléotide comprenant une séquence nucléotidique ayant une homologie d'au moins 55% avec la séquence nucléotidique indiquée dans l'un quelconque des : nucléotides 61 à 1539 de la SEQ ID n° : 1 ; nucléotides 58 à 1530 de la SEQ ID n° : 3 ; nucléotides 1 à 1479 de la SEQ ID n° : 5 ; ou nucléotides 1 à 1473 de la SEQ ID n° : 7.

2. Procédé selon la revendication 1, dans lequel l'oxydation accroît la sensibilité du matériel ligno-cellulosique à une dégradation enzymatique.

3. Procédé de production d'un sucre ou de sucres provenant d'un matériel ligno-cellulosique, lequel procédé comprend les étapes suivantes :
(i) un prétraitement du matériel ligno-cellulosique ;
(ii) une oxydation dudit matériel ligno-cellulosique en utilisant un procédé selon la revendication 1 ou la 2 ; et
(iii) une mise en contact du matériel ligno-cellulosique ainsi modifié avec une ou plusieurs cellulase(s) et/ou une ou plusieurs hémicellulase(s) et/ou une ou plusieurs pectinase(s).

4. Procédé selon la revendication 3, dans lequel le polypeptide, qui a une activité de peroxydase, est mis en contact avec le matériel qui comprend un glucide n'étant pas de l'amidon, pendant l'étape de prétraitement ou bien avant ou après celle-ci.

5. Procédé selon l'une quelconque des revendications précédentes, dans lequel le polypeptide, qui a une activité de peroxydase, est mis en contact avec le matériel ligno-cellulosique en combinaison avec une enzyme auxiliaire, laquelle enzyme auxiliaire modifie en outre ledit matériel, qui comprend un glucide n'étant pas de l'amidon, ou augmente la sensibilité dudit matériel, qui comprend un glucide n'étant pas de l'amidon, à une dégradation enzymatique, dans lequel l'enzyme auxiliaire est une enzyme des classes des : amylases, protéases, lipases, glucuronidases ou oxydoréductases.

6. Procédé selon l'une quelconque des revendications précédentes, dans lequel le polypeptide ayant une activité de peroxydase est codé par un polynucléotide que l'on peut obtenir à partir de *Marasmius scorodonius.*

7. Procédé selon l'une quelconque des revendications précédentes, dans lequel le matériel ligno-cellulosique est : issu de la préparation de vergers, un chaparral, des déchets d'usines, des déchets de bois urbains, des déchets municipaux, des résidus d'exploitations, du petit bois forestier, des cultures ligneuses à courte rotation, des déchets industriels, de la paille de blé, de la paille d'avoine, de la paille de riz, de la paille d'orge, de la paille de seigle, de la paille de lin, des écales de soja, des enveloppes du riz, des aliments à base de gluten de maïs, des enveloppes de l'avoine, de la canne à sucre, des cannes du maïs, des tiges du maïs, des rafles du maïs, des feuilles du maïs, une herbe des prairies, de l'herbe grama, une sétaire, de la pulpe de betterave sucrière, de la pulpe d'agrumes, des cosses de graines, des déchets animaux cellulosiques, des déchets de la tonte du gazon, du coton, des algues marines, des arbres, des arbustes, des herbes, du blé, de la bagasse de canne à sucre, du maïs, des grains de maïs, des fibres issues de graines, des produits et sous-produits provenant du broyage humide ou sec des grains, des déchets solides municipaux, des déchets du papier, des résidus de jardinage, un matériel herbacé, des résidus agricoles, des résidus forestiers, de la pulpe, des résidus d'usines à papier, des branches, des buissons, des cannes, une culture énergétique, la forêt, un fruit, une fleur, une semence, une culture herbacée, une feuille, de l'écorce, une aiguille, un rondin, une racine, un arbrisseau, un arbuste, le panic érigé, un légume, des écorces de fruits, une vigne, du son de blé, du bois dur ou mou, un matériel issu des déchets organiques généré à partir d'un processus agricole ou d'un déchet de bois de forêt, ou bien une combinaison de deux ou plusieurs éléments quelconques parmi ceux-ci.

8. Procédé de préparation d'un produit de fermentation, lequel procédé comprend les étapes consistant à :
a. traiter un matériel ligno-cellulosique, selon un procédé de l'une quelconque des revendications 1 à 7, et
b. fermenter le matériel résultant,
pour ainsi préparer un produit de fermentation.

9. Procédé selon la revendication 8, dans lequel le produit de fermentation est l'éthanol, le butanol, l'acide lactique, l'acide 3-hydroxy-propionique, l'acide acrylique, l'acide acétique, l'acide succinique, l'acide citrique, l'acide malique, l'acide fumarique, l'acide itaconique, un acide aminé, le 1,3-propane-diol, l'éthylène, le glycérol, un antibiotique à β-lactamine ou une céphalosporine.


### SEQUENCE LISTING

<110> DSM IP ASSETS B.V.
<120> METHOD FOR MODIFYING NON-STARCH CARBOHYDRATE MATERIAL
<130> 26485-WO-PCT
<160> 8
<170> PatentIn version 3.2
<210> 1
   <211> 1542
   <212> DNA
   <213> Marasmius scorodonius
<220>
   <221> CDS
   <222> (1)..(1542)
<400> 1
   atg aag ctt ttt tct gcc tct gtt ttt gct gct atc gtc gct agt cac        48
   Met Lys Leu Phe Ser Ala Ser Val Phe Ala Ala Ile Val Ala Ser His
   1               5                   10                  15
   tat gcg tca gcg act gcc cac atc agg gct ccc aat gtg aag cca agg        96
   Tyr Ala Ser Ala Thr Ala His Ile Arg Ala Pro Asn Val Lys Pro Arg
               20                  25                  30
   agg aca agc tca ctt ctg att act ccc cct caa cag cct ccg ctt cca       144
   Arg Thr Ser Ser Leu Leu Ile Thr Pro Pro Gln Gln Pro Pro Leu Pro
           35                  40                  45
   tct gct caa cag gct gca agt gcc tct agc agt gct ggc ttg aat ctc       192
   Ser Ala Gln Gln Ala Ala Ser Ala Ser Ser Ser Ala Gly Leu Asn Leu
       50                  55                  60
   acc gac att cag ggt gat att ctg atc ggc atg aag aag aac aag gaa       240
   Thr Asp Ile Gln Gly Asp Ile Leu Ile Gly Met Lys Lys Asn Lys Glu
   65                  70                  75                  80
   cta ttc ttc ttc ttc agt gtc acc gac gca gct act ttc aag gct aag       288
   Leu Phe Phe Phe Phe Ser Val Thr Asp Ala Ala Thr Phe Lys Ala Lys
                   85                  90                  95
   ctg gga tcc gac att ctt gga cta atc aca tcc acc gat cag cta ctt       336
   Leu Gly Ser Asp Ile Leu Gly Leu Ile Thr Ser Thr Asp Gln Leu Leu
               100                 105                 110
   gct aat gat act cag cct gtc acg gct gtc aac gtc gct ttc tct agc       384
   Ala Asn Asp Thr Gln Pro Val Thr Ala Val Asn Val Ala Phe Ser Ser
           115                 120                 125
   act ggc ctc aag gca ttg ggt atc aca gat ggt ctg aag gat cct gtc       432
   Thr Gly Leu Lys Ala Leu Gly Ile Thr Asp Gly Leu Lys Asp Pro Val
       130                 135                 140
   ttc gag gcc gga atg ctc agc aac gca gtg agc gac ttg agc gat cca      480
   Phe Glu Ala Gly Met Leu Ser Asn Ala Val Ser Asp Leu Ser Asp Pro
   145                 150                 155                 160
   ggg acc ggc aat tgg gtc cct ggg ttt gtc ggc acc agt gtt cat ggc      528
   Gly Thr Gly Asn Trp Val Pro Gly Phe Val Gly Thr Ser Val His Gly
                   165                 170                 175
   gtt ttc cta ctt gca tcg gac acc att gac aat gta aac acc gag ccg      576
   Val Phe Leu Leu Ala Ser Asp Thr Ile Asp Asn Val Asn Thr Glu Pro
               180                 185                 190
   gcc aac atc caa acc att ttg aat ggc tcg atc acg gag att cat cgt      624
   Ala Asn Ile Gln Thr Ile Leu Asn Gly Ser Ile Thr Glu Ile His Arg
           195                 200                 205
   tta caa ggg gag gct cga ccc ggt gac cag caa ggt cac gaa cac ttt      672
   Leu Gln Gly Glu Ala Arg Pro Gly Asp Gln Gln Gly His Glu His Phe
       210                 215                 220
   gga ttc atg gat gga atc agt aac ccg gcc gtt gat gga ttt aca cct      720
   Gly Phe Met Asp Gly Ile Ser Asn Pro Ala Val Asp Gly Phe Thr Pro
   225                 230                 235                 240
   cca gcg gaa ata aga cct gga caa gct tta att ccg cct ggt atc atg      768
   Pro Ala Glu Ile Arg Pro Gly Gln Ala Leu Ile Pro Pro Gly Ile Met
                   245                 250                 255
   ctt ctc gga gag gca aac gac act ttt cag aat gat cgt cct ccg tgg      816
   Leu Leu Gly Glu Ala Asn Asp Thr Phe Gln Asn Asp Arg Pro Pro Trp
               260                 265                 270
   gcc aaa gat ggt tcc ttc ctt gtc ttc cgt caa atg caa cag cgc gcg      864
   Ala Lys Asp Gly Ser Phe Leu Val Phe Arg Gln Met Gln Gln Arg Ala
           275                 280                 285
   ccc gag ttc aac aag ttc ctg caa gat cac gct ctt aac atg ccg aat      912
   Pro Glu Phe Asn Lys Phe Leu Gln Asp His Ala Leu Asn Met Pro Asn
       290                 295                 300
   atg aca tcc gag caa ggc gct gat ctc ctt ggt gcc agg att gta gga      960
   Met Thr Ser Glu Gln Gly Ala Asp Leu Leu Gly Ala Arg Ile Val Gly
   305                 310                 315                 320
   cga tgg aaa agt ggt gct cct att gac ctc act ccg ttg gtc gat gac     1008
   Arg Trp Lys Ser Gly Ala Pro Ile Asp Leu Thr Pro Leu Val Asp Asp
                   325                 330                 335
   cca gtg ttg gct gct gac aat cag cga aat aac aac ttc gac ttt tct     1056
   Pro Val Leu Ala Ala Asp Asn Gln Arg Asn Asn Asn Phe Asp Phe Ser
               340                 345                 350
   gac gcc acg aat cag aca cgt tgc cct ttc tct gct cat atc cgc aag     1104
   Asp Ala Thr Asn Gln Thr Arg Cys Pro Phe Ser Ala His Ile Arg Lys
           355                 360                 365
   gct aac ccg cgc ggc gat ctt ggg ggt att aat aaa ttc cca aac caa      1152
   Ala Asn Pro Arg Gly Asp Leu Gly Gly Ile Asn Lys Phe Pro Asn Gln
       370                 375                 380
   cac ata atc cga gcg gga att ccg tat gga ccc gaa gtt acc gac gct      1200
   His Ile Ile Arg Ala Gly Ile Pro Tyr Gly Pro Glu Val Thr Asp Ala
   385                 390                 395                 400
   gaa aga gcg tca aat agc tct agc act gac cct agt ctg gag cgt ggt      1248
   Glu Arg Ala Ser Asn Ser Ser Ser Thr Asp Pro Ser Leu Glu Arg Gly
                   405                 410                 415
   ctg gcg ttt gtg gcc tat cag tct aat atc cag aac gga ttc gta ttc      1296
   Leu Ala Phe Val Ala Tyr Gln Ser Asn Ile Gln Asn Gly Phe Val Phe
               420                 425                 430
   ctt caa aag aat tgg gtt gat aat acg aat ttc ttc cga ccc ggc act      1344
   Leu Gln Lys Asn Trp Val Asp Asn Thr Asn Phe Phe Arg Pro Gly Thr
           435                 440                 445
   ggt gca gat cct ctc atc ggt aca aat tct cgt aac agt ggc acc gat      1392
   Gly Ala Asp Pro Leu Ile Gly Thr Asn Ser Arg Asn Ser Gly Thr Asp
       450                 455                 460
   gcc ccc aac acg cct cgt gtc gtc agc ggc ttg gat cct aat aac gct      1440
   Ala Pro Asn Thr Pro Arg Val Val Ser Gly Leu Asp Pro Asn Asn Ala
   465                 470                 475                 480
   acg agc acc atc gaa att gat atc gat ttc gta gtt tct cgt gga gga      1488
   Thr Ser Thr Ile Glu Ile Asp Ile Asp Phe Val Val Ser Arg Gly Gly
                   485                 490                 495
   gaa tac ttc ttc tcg ccc tca ctt tct gcg atc agg act gtg ctt tca      1536
   Glu Tyr Phe Phe Ser Pro Ser Leu Ser Ala Ile Arg Thr Val Leu Ser
               500                 505                 510
   gtc tag                                                              1542
   Val
<210> 2
   <211> 513
   <212> PRT
   <213> Marasmius scorodonius
<400> 2
   Met Lys Leu Phe Ser Ala Ser Val Phe Ala Ala Ile Val Ala Ser His
   1               5                   10                  15
   Tyr Ala Ser Ala Thr Ala His Ile Arg Ala Pro Asn Val Lys Pro Arg
               20                  25                  30
   Arg Thr Ser Ser Leu Leu Ile Thr Pro Pro Gln Gln Pro Pro Leu Pro
           35                  40                  45
   Ser Ala Gln Gln Ala Ala Ser Ala Ser Ser Ser Ala Gly Leu Asn Leu
       50                  55                  60
   Thr Asp Ile Gln Gly Asp Ile Leu Ile Gly Met Lys Lys Asn Lys Glu
   65                  70                  75                  80
   Leu Phe Phe Phe Phe Ser Val Thr Asp Ala Ala Thr Phe Lys Ala Lys
                   85                  90                  95
   Leu Gly Ser Asp Ile Leu Gly Leu Ile Thr Ser Thr Asp Gln Leu Leu
               100                 105                 110
   Ala Asn Asp Thr Gln Pro Val Thr Ala Val Asn Val Ala Phe Ser Ser
           115                 120                 125
   Thr Gly Leu Lys Ala Leu Gly Ile Thr Asp Gly Leu Lys Asp Pro Val
       130                 135                 140
   Phe Glu Ala Gly Met Leu Ser Asn Ala Val Ser Asp Leu Ser Asp Pro
   145                 150                 155                 160
   Gly Thr Gly Asn Trp Val Pro Gly Phe Val Gly Thr Ser Val His Gly
                   165                 170                 175
   Val Phe Leu Leu Ala Ser Asp Thr Ile Asp Asn Val Asn Thr Glu Pro
               180                 185                 190
   Ala Asn Ile Gln Thr Ile Leu Asn Gly Ser Ile Thr Glu Ile His Arg
           195                 200                 205
   Leu Gln Gly Glu Ala Arg Pro Gly Asp Gln Gln Gly His Glu His Phe
       210                 215                 220
   Gly Phe Met Asp Gly Ile Ser Asn Pro Ala Val Asp Gly Phe Thr Pro
   225                 230                 235                 240
   Pro Ala Glu Ile Arg Pro Gly Gln Ala Leu Ile Pro Pro Gly Ile Met
                   245                 250                 255
   Leu Leu Gly Glu Ala Asn Asp Thr Phe Gln Asn Asp Arg Pro Pro Trp
   260 265 270
   Ala Lys Asp Gly Ser Phe Leu Val Phe Arg Gln Met Gln Gln Arg Ala
   275 280 285
   Pro Glu Phe Asn Lys Phe Leu Gln Asp His Ala Leu Asn Met Pro Asn
   290 295 300
   Met Thr Ser Glu Gln Gly Ala Asp Leu Leu Gly Ala Arg Ile Val Gly
   305 310 315 320
   Arg Trp Lys Ser Gly Ala Pro Ile Asp Leu Thr Pro Leu Val Asp Asp
   325 330 335
   Pro Val Leu Ala Ala Asp Asn Gln Arg Asn Asn Asn Phe Asp Phe Ser
   340 345 350
   Asp Ala Thr Asn Gln Thr Arg Cys Pro Phe Ser Ala His Ile Arg Lys
   355 360 365
   Ala Asn Pro Arg Gly Asp Leu Gly Gly Ile Asn Lys Phe Pro Asn Gln
   370 375 380
   His Ile Ile Arg Ala Gly Ile Pro Tyr Gly Pro Glu Val Thr Asp Ala
   385 390 395 400
   Glu Arg Ala Ser Asn Ser Ser Ser Thr Asp Pro Ser Leu Glu Arg Gly
   405 410 415
   Leu Ala Phe Val Ala Tyr Gln Ser Asn Ile Gln Asn Gly Phe Val Phe
   420 425 430
   Leu Gln Lys Asn Trp Val Asp Asn Thr Asn Phe Phe Arg Pro Gly Thr
   435 440 445
   Gly Ala Asp Pro Leu Ile Gly Thr Asn Ser Arg Asn Ser Gly Thr Asp
   450 455 460
   Ala Pro Asn Thr Pro Arg Val Val Ser Gly Leu Asp Pro Asn Asn Ala
   465 470 475 480
   Thr Ser Thr Ile Glu Ile Asp Ile Asp Phe Val Val Ser Arg Gly Gly
   485 490 495
   Glu Tyr Phe Phe Ser Pro Ser Leu Ser Ala Ile Arg Thr Val Leu Ser
               500                 505                 510
   Val
<210> 3
   <211> 1533
   <212> DNA
   <213> Marasmius scorodonius
<220>
   <221> CDS
   <222> (1)..(1533)
<400> 3
   atg cgg ctc act tac ctt ccc ttg ttt gcg ggc atc gcc atc cag tct       48
   Met Arg Leu Thr Tyr Leu Pro Leu Phe Ala Gly Ile Ala Ile Gln Ser
   1               5                   10                  15
   gca tgt gcc ttt cca aac ttc tcc aag tct tcc ata tta aag cct cgt       96
   Ala Cys Ala Phe Pro Asn Phe Ser Lys Ser Ser Ile Leu Lys Pro Arg
               20                  25                  30
   agg acg aac tcc cta cta atc aat ccc gat gct cag cca gac ctc ccg      144
   Arg Thr Asn Ser Leu Leu Ile Asn Pro Asp Ala Gln Pro Asp Leu Pro
           35                  40                  45
   acc gca aag cag gct tcc act gcg gct gct tct gtg ggt ttg aac ctt      192
   Thr Ala Lys Gln Ala Ser Thr Ala Ala Ala Ser Val Gly Leu Asn Leu
       50                  55                  60
   acc gac atc caa gga gac atc tta atc ggt atg aag aag aac aag gaa      240
   Thr Asp Ile Gln Gly Asp Ile Leu Ile Gly Met Lys Lys Asn Lys Glu
   65                  70                  75                  80
   atg ttc ttc ttc ttc agt att gca gat gcc gct gca ttc aaa tcc cac      288
   Met Phe Phe Phe Phe Ser Ile Ala Asp Ala Ala Ala Phe Lys Ser His
                   85                  90                  95
   ttg ggc tcc gct att ctc cct ctt atc acc tcg acg caa cag ctg ctt      336
   Leu Gly Ser Ala Ile Leu Pro Leu Ile Thr Ser Thr Gln Gln Leu Leu
               100                 105                 110
   gct gtt gcc agc cag cct acc acc gct gtc aac ctt gcc ttc tcc caa      384
   Ala Val Ala Ser Gln Pro Thr Thr Ala Val Asn Leu Ala Phe Ser Gln
           115                 120                 125
   acc gga ttg aat gcg ctg ggg ctt gct gct caa ggc ttg ggg gac tcc      432
   Thr Gly Leu Asn Ala Leu Gly Leu Ala Ala Gln Gly Leu Gly Asp Ser
       130                 135                 140
   ctc ttt gcc agt ggt cag ttc agc ggt gca cag agt ctc ggc gac ccg      480
   Leu Phe Ala Ser Gly Gln Phe Ser Gly Ala Gln Ser Leu Gly Asp Pro
   145                 150                 155                 160
   gga acc tcg aac tgg gtc caa gcg ttc gct ggt aca ggc ata cat ggt      528
   Gly Thr Ser Asn Trp Val Gln Ala Phe Ala Gly Thr Gly Ile His Gly
                   165                 170                 175
   gtc ttc ctt ctc gca tcg gat acc gtc gac aac gtg aat gcc gag ctg      576
   Val Phe Leu Leu Ala Ser Asp Thr Val Asp Asn Val Asn Ala Glu Leu
               180                 185                 190
   tcg caa atc cag tcc atc ttg gga acc tcc atc act gag gcg tac cgc      624
   Ser Gln Ile Gln Ser Ile Leu Gly Thr Ser Ile Thr Glu Ala Tyr Arg
           195                 200                 205
   ctt cag ggt gag gct cgt ccc ggc gat cag caa ggc cac gaa cat ttc      672
   Leu Gln Gly Glu Ala Arg Pro Gly Asp Gln Gln Gly His Glu His Phe
       210                 215                 220
   gga ttc atg gac gga atc agc aac cct gcc atc gac gga ttc tcc act      720
   Gly Phe Met Asp Gly Ile Ser Asn Pro Ala Ile Asp Gly Phe Ser Thr
   225                 230                 235                 240
   gcg ctg cct ggt caa gcc gtt ctc agt ccc gga ctt ttc ctg cta gga      768
   Ala Leu Pro Gly Gln Ala Val Leu Ser Pro Gly Leu Phe Leu Leu Gly
                   245                 250                 255
   gag gac ggc gat ggt tcc tcg tct tcg cgt ccg tct tgg gca aag gac      816
   Glu Asp Gly Asp Gly Ser Ser Ser Ser Arg Pro Ser Trp Ala Lys Asp
               260                 265                 270
   ggc tct ttc ctt gct ttc cgc cag ctt caa cag cgt gtc cca gag ttc      864
   Gly Ser Phe Leu Ala Phe Arg Gln Leu Gln Gln Arg Val Pro Glu Phe
           275                 280                 285
   aac aag ttc ctc gct gac aac gct gcg cta aca cag ggt aac gct gat      912
   Asn Lys Phe Leu Ala Asp Asn Ala Ala Leu Thr Gln Gly Asn Ala Asp
       290                 295                 300
   ctt ctc ggt gcc cga atg atg gga cgg tgg aaa tct ggt gct ccg gtc      960
   Leu Leu Gly Ala Arg Met Met Gly Arg Trp Lys Ser Gly Ala Pro Val
   305                 310                 315                 320
   gac ctt gcc ccc acc gcg gac gat gtt gat ctc gcc aat gac ccc caa     1008
   Asp Leu Ala Pro Thr Ala Asp Asp Val Asp Leu Ala Asn Asp Pro Gln
                   325                 330                 335
   agg aac aac aac ttc aac ttt acc cac gcc ggt ttc acc gag acc act     1056
   Arg Asn Asn Asn Phe Asn Phe Thr His Ala Gly Phe Thr Glu Thr Thr
               340                 345                 350
   gac gaa act cac tgc ccc ttc tcc gct cac atc cgt aag acg aac cct     1104
   Asp Glu Thr His Cys Pro Phe Ser Ala His Ile Arg Lys Thr Asn Pro
           355                 360                 365
   cga tcg gac ttc aac ccc cag aac acc aac aac cac atc atc cgt gct     1152
   Arg Ser Asp Phe Asn Pro Gln Asn Thr Asn Asn His Ile Ile Arg Ala
       370                 375                 380
   ggt att cct tac gga cct gaa gtc act gac gct gaa gcc tca tcc aac      1200
   Gly Ile Pro Tyr Gly Pro Glu Val Thr Asp Ala Glu Ala Ser Ser Asn
   385                 390                 395                 400
   acg tcc agc acg gac gct agc ctc gag cgt ggc ttg gct ttc gtt gca      1248
   Thr Ser Ser Thr Asp Ala Ser Leu Glu Arg Gly Leu Ala Phe Val Ala
                   405                 410                 415
   tac caa tcg aac att ggc aac ggc ttt gca ttc att caa cag aat tgg      1296
   Tyr Gln Ser Asn Ile Gly Asn Gly Phe Ala Phe Ile Gln Gln Asn Trp
               420                 425                 430
   gtt gac aac gca aac ttc ttc ttc gga aaa acc acc ccg cct ggt att      1344
   Val Asp Asn Ala Asn Phe Phe Phe Gly Lys Thr Thr Pro Pro Gly Ile
           435                 440                 445
   gac ccc atc atc ggc tcg aat gct gca cag aac aac ttc gct ccc aac      1392
   Asp Pro Ile Ile Gly Ser Asn Ala Ala Gln Asn Asn Phe Ala Pro Asn
       450                 455                 460
   tct cca cgt cct gtg tcc gga ctt gac ccc aca gat tcg acc acg atc      1440
   Ser Pro Arg Pro Val Ser Gly Leu Asp Pro Thr Asp Ser Thr Thr Ile
   465                 470                 475                 480
   gtc acc tta aac acc gac ttc gtt gtt tct cgt gga gga gag tac ttc      1488
   Val Thr Leu Asn Thr Asp Phe Val Val Ser Arg Gly Gly Glu Tyr Phe
                   485                 490                 495
   ttc tcc ccc tcg ctc tct gcg atc cag aac acg ctt tct gtt tga          1533
   Phe Ser Pro Ser Leu Ser Ala Ile Gln Asn Thr Leu Ser Val
               500                 505                 510
<210> 4
   <211> 510
   <212> PRT
   <213> Marasmius scorodonius
<400> 4
   Met Arg Leu Thr Tyr Leu Pro Leu Phe Ala Gly Ile Ala Ile Gln Ser
   1               5                   10                  15
   Ala Cys Ala Phe Pro Asn Phe Ser Lys Ser Ser Ile Leu Lys Pro Arg
               20                  25                  30
   Arg Thr Asn Ser Leu Leu Ile Asn Pro Asp Ala Gln Pro Asp Leu Pro
           35                  40                  45
   Thr Ala Lys Gln Ala Ser Thr Ala Ala Ala Ser Val Gly Leu Asn Leu
       50                  55                  60
   Thr Asp Ile Gln Gly Asp Ile Leu Ile Gly Met Lys Lys Asn Lys Glu
   65                  70                  75                  80
   Met Phe Phe Phe Phe Ser Ile Ala Asp Ala Ala Ala Phe Lys Ser His
                   85                  90                  95
   Leu Gly Ser Ala Ile Leu Pro Leu Ile Thr Ser Thr Gln Gln Leu Leu
               100                 105                 110
   Ala Val Ala Ser Gln Pro Thr Thr Ala Val Asn Leu Ala Phe Ser Gln
           115                 120                 125
   Thr Gly Leu Asn Ala Leu Gly Leu Ala Ala Gln Gly Leu Gly Asp Ser
       130                 135                 140
   Leu Phe Ala Ser Gly Gln Phe Ser Gly Ala Gln Ser Leu Gly Asp Pro
   145                 150                 155                 160
   Gly Thr Ser Asn Trp Val Gln Ala Phe Ala Gly Thr Gly Ile His Gly
                   165                 170                 175
   Val Phe Leu Leu Ala Ser Asp Thr Val Asp Asn Val Asn Ala Glu Leu
               180                 185                 190
   Ser Gln Ile Gln Ser Ile Leu Gly Thr Ser Ile Thr Glu Ala Tyr Arg
           195                 200                 205
   Leu Gln Gly Glu Ala Arg Pro Gly Asp Gln Gln Gly His Glu His Phe
       210                 215                 220
   Gly Phe Met Asp Gly Ile Ser Asn Pro Ala Ile Asp Gly Phe Ser Thr
   225                 230                 235                 240
   Ala Leu Pro Gly Gln Ala Val Leu Ser Pro Gly Leu Phe Leu Leu Gly
                   245                 250                 255
   Glu Asp Gly Asp Gly Ser Ser Ser Ser Arg Pro Ser Trp Ala Lys Asp
               260                 265                 270
   Gly Ser Phe Leu Ala Phe Arg Gln Leu Gln Gln Arg Val Pro Glu Phe
           275                 280                 285
   Asn Lys Phe Leu Ala Asp Asn Ala Ala Leu Thr Gln Gly Asn Ala Asp
       290                 295                 300
   Leu Leu Gly Ala Arg Met Met Gly Arg Trp Lys Ser Gly Ala Pro Val
   305                 310                 315                 320
   Asp Leu Ala Pro Thr Ala Asp Asp Val Asp Leu Ala Asn Asp Pro Gln
                   325                 330                 335
   Arg Asn Asn Asn Phe Asn Phe Thr His Ala Gly Phe Thr Glu Thr Thr
               340                 345                 350
   Asp Glu Thr His Cys Pro Phe Ser Ala His Ile Arg Lys Thr Asn Pro
           355                 360                 365
   Arg Ser Asp Phe Asn Pro Gln Asn Thr Asn Asn His Ile Ile Arg Ala
       370                 375                 380
   Gly Ile Pro Tyr Gly Pro Glu Val Thr Asp Ala Glu Ala Ser Ser Asn
   385                 390                 395                 400
   Thr Ser Ser Thr Asp Ala Ser Leu Glu Arg Gly Leu Ala Phe Val Ala
                   405                 410                 415
   Tyr Gln Ser Asn Ile Gly Asn Gly Phe Ala Phe Ile Gln Gln Asn Trp
               420                 425                 430
   Val Asp Asn Ala Asn Phe Phe Phe Gly Lys Thr Thr Pro Pro Gly Ile
           435                 440                 445
   Asp Pro Ile Ile Gly Ser Asn Ala Ala Gln Asn Asn Phe Ala Pro Asn
       450                 455                 460
   Ser Pro Arg Pro Val Ser Gly Leu Asp Pro Thr Asp Ser Thr Thr Ile
   465                 470                 475                 480
   Val Thr Leu Asn Thr Asp Phe Val Val Ser Arg Gly Gly Glu Tyr Phe
                   485                 490                 495
   Phe Ser Pro Ser Leu Ser Ala Ile Gln Asn Thr Leu Ser Val
               500                 505                 510
<210> 5
   <211> 1482
   <212> DNA
   <213> Marasmius scorodonius
<220>
   <221> CDS
   <222> (1)..(1482)
<400> 5
   act gcc cac atc cgc gct ccc aac gtg aag ccc cgc cgc acc aac tcc        48
   Thr Ala His Ile Arg Ala Pro Asn Val Lys Pro Arg Arg Thr Asn Ser
   1               5                   10                  15
   ctt ctg atc act ccc cct cag cag cct ccc ctt ccc tct gct cag cag        96
   Leu Leu Ile Thr Pro Pro Gln Gln Pro Pro Leu Pro Ser Ala Gln Gln
               20                  25                  30
   gct gcc tcc gcc tcc agc tcc gct ggc ctc aac ctt acc gac att cag       144
   Ala Ala Ser Ala Ser Ser Ser Ala Gly Leu Asn Leu Thr Asp Ile Gln
           35                  40                  45
   ggt gac att ctg atc ggc atg aag aag aac aag gaa ctc ttc ttc ttc       192
   Gly Asp Ile Leu Ile Gly Met Lys Lys Asn Lys Glu Leu Phe Phe Phe
       50                  55                  60
   ttc tcc gtc acc gac gcc gct act ttc aag gct aag ctc gga tcc gac       240
   Phe Ser Val Thr Asp Ala Ala Thr Phe Lys Ala Lys Leu Gly Ser Asp
   65                  70                  75                  80
   att ctc ggt ctg atc acc tcc acc gat cag ctg ctc gct aac gac act       288
   Ile Leu Gly Leu Ile Thr Ser Thr Asp Gln Leu Leu Ala Asn Asp Thr
                   85                  90                  95
   cag cct gtc acc gct gtc aac gtc gct ttc tct agc act ggc ctc aag       336
   Gln Pro Val Thr Ala Val Asn Val Ala Phe Ser Ser Thr Gly Leu Lys
               100                 105                 110
   gcc ttg ggt atc acc gac gat ctg aag gat ccc gtc ttc gag gcc ggt       384
   Ala Leu Gly Ile Thr Asp Asp Leu Lys Asp Pro Val Phe Glu Ala Gly
           115                 120                 125
   atg ctc agc aac gcc gtg agc gac ttg agc gat ccc ggt acc ggc aac       432
   Met Leu Ser Asn Ala Val Ser Asp Leu Ser Asp Pro Gly Thr Gly Asn
       130                 135                 140
   tgg gtc cct ggc ttc gtc ggc acc tcc gtt cac ggc gtt ttc ctg ctc       480
   Trp Val Pro Gly Phe Val Gly Thr Ser Val His Gly Val Phe Leu Leu
   145                 150                 155                 160
   gcc tcg gac acc atc gac aac gtc aac acc gag ctg gcc aac atc cag       528
   Ala Ser Asp Thr Ile Asp Asn Val Asn Thr Glu Leu Ala Asn Ile Gln
                   165                 170                 175
   acc att ttg aac ggc tcg atc acc gag att cac cgt ctg cag ggt gag       576
   Thr Ile Leu Asn Gly Ser Ile Thr Glu Ile His Arg Leu Gln Gly Glu
               180                 185                 190
   gct cgt ccc ggt gac cag cag ggt cac gag cac ttc gga ttc atg gac      624
   Ala Arg Pro Gly Asp Gln Gln Gly His Glu His Phe Gly Phe Met Asp
           195                 200                 205
   ggt atc tcc aac ccc gcc gtt gac ggc ttc acc cct ccc gcg gaa atc      672
   Gly Ile Ser Asn Pro Ala Val Asp Gly Phe Thr Pro Pro Ala Glu Ile
       210                 215                 220
   cgt cct gga cag gct ctc att ccc ccc ggt atc atg ctt ctc ggt gag      720
   Arg Pro Gly Gln Ala Leu Ile Pro Pro Gly Ile Met Leu Leu Gly Glu
   225                 230                 235                 240
   gcc aac gac act ttc cag aac gat cgt cct ccc tgg gcc aag gac ggt      768
   Ala Asn Asp Thr Phe Gln Asn Asp Arg Pro Pro Trp Ala Lys Asp Gly
                   245                 250                 255
   tcc ttc ctt gtc ttc cgt cag atg cag cag cgc gcg ccc gag ttc aac      816
   Ser Phe Leu Val Phe Arg Gln Met Gln Gln Arg Ala Pro Glu Phe Asn
               260                 265                 270
   aag ttc ctg cag gat cac gcc ctc aac atg ccc aac atg acc tcc gag      864
   Lys Phe Leu Gln Asp His Ala Leu Asn Met Pro Asn Met Thr Ser Glu
           275                 280                 285
   cag ggc gct gat ctc ctt ggt gcc cgc att gtt gga cgc tgg aag tcc      912
   Gln Gly Ala Asp Leu Leu Gly Ala Arg Ile Val Gly Arg Trp Lys Ser
       290                 295                 300
   ggt gct cct atc gac ctc act ccc ttg gtc gat gac ccc gtg ttg gcc      960
   Gly Ala Pro Ile Asp Leu Thr Pro Leu Val Asp Asp Pro Val Leu Ala
   305                 310                 315                 320
   gcc gac aac cag cgc aac aac aac ttc gac ttc tct gac gcc acc aac     1008
   Ala Asp Asn Gln Arg Asn Asn Asn Phe Asp Phe Ser Asp Ala Thr Asn
                   325                 330                 335
   cag acc cgt tgc cct ttc tct gct cac atc cgc aag gct aac ccc cgc     1056
   Gln Thr Arg Cys Pro Phe Ser Ala His Ile Arg Lys Ala Asn Pro Arg
               340                 345                 350
   ggc gat ctc ggt ggt atc aac aag ttc ccc aac cag cac atc atc cgc     1104
   Gly Asp Leu Gly Gly Ile Asn Lys Phe Pro Asn Gln His Ile Ile Arg
           355                 360                 365
   gcc ggt att ccc tac ggt ccc gaa gtt acc gac gct gag aag gcg tcc     1152
   Ala Gly Ile Pro Tyr Gly Pro Glu Val Thr Asp Ala Glu Lys Ala Ser
       370                 375                 380
   aac agc tct agc act gac cct tcg ctg gag cgt ggt ctg gcg ttc gtg     1200
   Asn Ser Ser Ser Thr Asp Pro Ser Leu Glu Arg Gly Leu Ala Phe Val
   385                 390                 395                 400
   gcc tac cag tct aac atc cag aac gga ttc gtg ttc ctt cag aag aac     1248
   Ala Tyr Gln Ser Asn Ile Gln Asn Gly Phe Val Phe Leu Gln Lys Asn
                   405                 410                 415
   tgg gtt gac aac acc aac ttc ttc cgt ccc ggc act ggt gtc gat cct      1296
   Trp Val Asp Asn Thr Asn Phe Phe Arg Pro Gly Thr Gly Val Asp Pro
               420                 425                 430
   ctc atc ggt acc aac tct cgt aac tcc ggc acc gat gcc ccc aac acc      1344
   Leu Ile Gly Thr Asn Ser Arg Asn Ser Gly Thr Asp Ala Pro Asn Thr
           435                 440                 445
   cct cgt gtc gtc agc ggc ttg gat cct aac aac gct acc agc acc atc      1392
   Pro Arg Val Val Ser Gly Leu Asp Pro Asn Asn Ala Thr Ser Thr Ile
       450                 455                 460
   gag atc gat atc gac ttc gtc gtt tct cgt gga ggc gaa tac ttc ttc      1440
   Glu Ile Asp Ile Asp Phe Val Val Ser Arg Gly Gly Glu Tyr Phe Phe
   465                 470                 475                 480
   tcg ccc tcg ctc tct gcc atc cgc act gtg ctc tcc gtc taa              1482
   Ser Pro Ser Leu Ser Ala Ile Arg Thr Val Leu Ser Val
                   485                 490
<210> 6
   <211> 493
   <212> PRT
   <213> Marasmius scorodonius
<400> 6
   Thr Ala His Ile Arg Ala Pro Asn Val Lys Pro Arg Arg Thr Asn Ser
   1               5                   10                  15
   Leu Leu Ile Thr Pro Pro Gln Gln Pro Pro Leu Pro Ser Ala Gln Gln
               20                  25                  30
   Ala Ala Ser Ala Ser Ser Ser Ala Gly Leu Asn Leu Thr Asp Ile Gln
           35                  40                  45
   Gly Asp Ile Leu Ile Gly Met Lys Lys Asn Lys Glu Leu Phe Phe Phe
       50                  55                  60
   Phe Ser Val Thr Asp Ala Ala Thr Phe Lys Ala Lys Leu Gly Ser Asp
   65                  70                  75                  80
   Ile Leu Gly Leu Ile Thr Ser Thr Asp Gln Leu Leu Ala Asn Asp Thr
                   85                  90                  95
   Gln Pro Val Thr Ala Val Asn Val Ala Phe Ser Ser Thr Gly Leu Lys
               100                 105                 110
   Ala Leu Gly Ile Thr Asp Asp Leu Lys Asp Pro Val Phe Glu Ala Gly
           115                 120                 125
   Met Leu Ser Asn Ala Val Ser Asp Leu Ser Asp Pro Gly Thr Gly Asn
       130                 135                 140
   Trp Val Pro Gly Phe Val Gly Thr Ser Val His Gly Val Phe Leu Leu
   145                 150                 155                 160
   Ala Ser Asp Thr Ile Asp Asn Val Asn Thr Glu Leu Ala Asn Ile Gln
                   165                 170                 175
   Thr Ile Leu Asn Gly Ser Ile Thr Glu Ile His Arg Leu Gln Gly Glu
               180                 185                 190
   Ala Arg Pro Gly Asp Gln Gln Gly His Glu His Phe Gly Phe Met Asp
           195                 200                 205
   Gly Ile Ser Asn Pro Ala Val Asp Gly Phe Thr Pro Pro Ala Glu Ile
       210                 215                 220
   Arg Pro Gly Gln Ala Leu Ile Pro Pro Gly Ile Met Leu Leu Gly Glu
   225                 230                 235                 240
   Ala Asn Asp Thr Phe Gln Asn Asp Arg Pro Pro Trp Ala Lys Asp Gly
                   245                 250                 255
   Ser Phe Leu Val Phe Arg Gln Met Gln Gln Arg Ala Pro Glu Phe Asn
               260                 265                 270
   Lys Phe Leu Gln Asp His Ala Leu Asn Met Pro Asn Met Thr Ser Glu
           275                 280                 285
   Gln Gly Ala Asp Leu Leu Gly Ala Arg Ile Val Gly Arg Trp Lys Ser
       290                 295                 300
   Gly Ala Pro Ile Asp Leu Thr Pro Leu Val Asp Asp Pro Val Leu Ala
   305                 310                 315                 320
   Ala Asp Asn Gln Arg Asn Asn Asn Phe Asp Phe Ser Asp Ala Thr Asn
                   325                 330                 335
   Gln Thr Arg Cys Pro Phe Ser Ala His Ile Arg Lys Ala Asn Pro Arg
                   340                 345                 350
   Gly Asp Leu Gly Gly Ile Asn Lys Phe Pro Asn Gln His Ile Ile Arg
           355                 360                 365
   Ala Gly Ile Pro Tyr Gly Pro Glu Val Thr Asp Ala Glu Lys Ala Ser
       370                 375                 380
   Asn Ser Ser Ser Thr Asp Pro Ser Leu Glu Arg Gly Leu Ala Phe Val
   385                 390                 395                 400
   Ala Tyr Gln Ser Asn Ile Gln Asn Gly Phe Val Phe Leu Gln Lys Asn
                   405                 410                 415
   Trp Val Asp Asn Thr Asn Phe Phe Arg Pro Gly Thr Gly Val Asp Pro
               420                 425                 430
   Leu Ile Gly Thr Asn Ser Arg Asn Ser Gly Thr Asp Ala Pro Asn Thr
           435                 440                 445
   Pro Arg Val Val Ser Gly Leu Asp Pro Asn Asn Ala Thr Ser Thr Ile
       450                 455                 460
   Glu Ile Asp Ile Asp Phe Val Val Ser Arg Gly Gly Glu Tyr Phe Phe
   465                 470                 475                 480
   Ser Pro Ser Leu Ser Ala Ile Arg Thr Val Leu Ser Val
                   485                 490
<210> 7
   <211> 1476
   <212> DNA
   <213> Marasmius scorodonius
<220>
   <221> CDS
   <222> (1)..(1476)
<400> 7
   ttc cct aac ttc tcc aag tct agc atc ctc aag ccc cgt cgt act aac      48
   Phe Pro Asn Phe Ser Lys Ser Ser Ile Leu Lys Pro Arg Arg Thr Asn
   1               5                   10                  15
   tct ctt ctg atc aac ccc gac gct cag ccc gat ttg ccc acc gcg aag      96
   Ser Leu Leu Ile Asn Pro Asp Ala Gln Pro Asp Leu Pro Thr Ala Lys
               20                  25                  30
   cag gct tct act gcc gcc gcc tcc gtt gga ctc aac ttg acc gac att        144
   Gln Ala Ser Thr Ala Ala Ala Ser Val Gly Leu Asn Leu Thr Asp Ile
           35                  40                  45
   cag ggc gat att ctc atc ggt atg aag aag aac aag gaa atg ttc ttc        192
   Gln Gly Asp Ile Leu Ile Gly Met Lys Lys Asn Lys Glu Met Phe Phe
       50                  55                  60
   ttc ttc tcc att gcc gat gct gcc gct ttc aag agc cac ctc ggt tcg        240
   Phe Phe Ser Ile Ala Asp Ala Ala Ala Phe Lys Ser His Leu Gly Ser
   65                  70                  75                  80
   gcc att ctc ccc ctt atc acc agc act cag cag ctt ctg gct gtt gcc        288
   Ala Ile Leu Pro Leu Ile Thr Ser Thr Gln Gln Leu Leu Ala Val Ala
                   85                  90                  95
   tcc cag cct acc act gcg gtt aac ttg gcc ttc tcc cag acc ggc ctc        336
   Ser Gln Pro Thr Thr Ala Val Asn Leu Ala Phe Ser Gln Thr Gly Leu
               100                 105                 110
   aac gcg ctg ggc ctc gct gcc cag ggt ctt gga gac tcc ctg ttc gct        384
   Asn Ala Leu Gly Leu Ala Ala Gln Gly Leu Gly Asp Ser Leu Phe Ala
           115                 120                 125
   tct ggt cag ttc tct ggc gcc cag tcc ctg gga gat cct ggt act tcg        432
   Ser Gly Gln Phe Ser Gly Ala Gln Ser Leu Gly Asp Pro Gly Thr Ser
       130                 135                 140
   aac tgg gtc cag gcc ttc gct ggc acc ggt atc cac ggt gtc ttc ctc        480
   Asn Trp Val Gln Ala Phe Ala Gly Thr Gly Ile His Gly Val Phe Leu
   145                 150                 155                 160
   ttg gct tcc gat acc gtt gac aac gtg aac gcc gag ctc agc cag atc        528
   Leu Ala Ser Asp Thr Val Asp Asn Val Asn Ala Glu Leu Ser Gln Ile
                   165                 170                 175
   cag tcc atc ctc ggc acc tcc att act gag gct tac cgc ctc cag gga        576
   Gln Ser Ile Leu Gly Thr Ser Ile Thr Glu Ala Tyr Arg Leu Gln Gly
               180                 185                 190
   gag gcg cgt ccc ggt gac cag cag ggt cac gag cac ttc ggc ttc atg        624
   Glu Ala Arg Pro Gly Asp Gln Gln Gly His Glu His Phe Gly Phe Met
           195                 200                 205
   gac ggc atc tcg aac cct gcc atc gac ggt ttc agc acc gct ctg ccc        672
   Asp Gly Ile Ser Asn Pro Ala Ile Asp Gly Phe Ser Thr Ala Leu Pro
       210                 215                 220
   ggc cag gct gtg ctt tcc ccc ggc ctg ttc ctg ttg ggt gaa gac ggt        720
   Gly Gln Ala Val Leu Ser Pro Gly Leu Phe Leu Leu Gly Glu Asp Gly
   225                 230                 235                 240
   gat ggt agc tcg tcc tct cgc ccc agc tgg gct aag gac gga tcc ttc        768
   Asp Gly Ser Ser Ser Ser Arg Pro Ser Trp Ala Lys Asp Gly Ser Phe
                   245                 250                 255
   ctc gcc ttc cgt cag ctc cag cag cgt gtt ccc gag ttc aac aag ttc      816
   Leu Ala Phe Arg Gln Leu Gln Gln Arg Val Pro Glu Phe Asn Lys Phe
               260                 265                 270
   ctc gcc gac aac gct gcc ctg acc cag ggt aac gcc gat ctt ctc gga      864
   Leu Ala Asp Asn Ala Ala Leu Thr Gln Gly Asn Ala Asp Leu Leu Gly
           275                 280                 285
   gcc cgc atg atg ggc cgc tgg aag tct ggt gcc ccc gtc gac ctg gct      912
   Ala Arg Met Met Gly Arg Trp Lys Ser Gly Ala Pro Val Asp Leu Ala
       290                 295                 300
   cct acc gcg gac gat gtg gac ttg gcc aac gac cct cag cgc aac aac      960
   Pro Thr Ala Asp Asp Val Asp Leu Ala Asn Asp Pro Gln Arg Asn Asn
   305                 310                 315                 320
   aac ttc aac ttc acc cac gcc ggc ttc acc gag acc acc gac gag act     1008
   Asn Phe Asn Phe Thr His Ala Gly Phe Thr Glu Thr Thr Asp Glu Thr
                   325                 330                 335
   cac tgc ccc ttc tcc gcc cac atc cgc aag acc aac ccc cgt tcc gat     1056
   His Cys Pro Phe Ser Ala His Ile Arg Lys Thr Asn Pro Arg Ser Asp
               340                 345                 350
   ttc aac cct cag aac act aac aac cac atc atc cgt gct ggt atc cct     1104
   Phe Asn Pro Gln Asn Thr Asn Asn His Ile Ile Arg Ala Gly Ile Pro
           355                 360                 365
   tac ggt ccc gaa gtc acc gac gct gag gcc tct tcg aac acc tcc tcc     1152
   Tyr Gly Pro Glu Val Thr Asp Ala Glu Ala Ser Ser Asn Thr Ser Ser
       370                 375                 380
   acc gac gct tcc ctc gag cgc ggc ctt gcc ttc gtc gcg tac cag agc     1200
   Thr Asp Ala Ser Leu Glu Arg Gly Leu Ala Phe Val Ala Tyr Gln Ser
   385                 390                 395                 400
   aac att ggt aac ggc ttc gct ttc ctg cag cag gcc tgg gtt gat aac     1248
   Asn Ile Gly Asn Gly Phe Ala Phe Leu Gln Gln Ala Trp Val Asp Asn
                   405                 410                 415
   gcc aac ttc ttc ttc ggc aag acc act ccc ccc gga gtg gac ccc atc     1296
   Ala Asn Phe Phe Phe Gly Lys Thr Thr Pro Pro Gly Val Asp Pro Ile
               420                 425                 430
   att ggt tct gtg gct gcc cag aac aac ttc gct cct aac ggt ccc cgt     1344
   Ile Gly Ser Val Ala Ala Gln Asn Asn Phe Ala Pro Asn Gly Pro Arg
           435                 440                 445
   ccc gtt agc ggt ctc gac cct acc gat tcc act aag atc gtc acc atc     1392
   Pro Val Ser Gly Leu Asp Pro Thr Asp Ser Thr Lys Ile Val Thr Ile
       450                 455                 460
   aac acc gac ttc gtg tcc tcg cgc ggc ggt gag tac ttc ttc agc ccc     1440
   Asn Thr Asp Phe Val Ser Ser Arg Gly Gly Glu Tyr Phe Phe Ser Pro
   465                 470                 475                 480
   tct ctg tcc gcc atc cag aac acc ctc tcc gtc taa                     1476
   Ser Leu Ser Ala Ile Gln Asn Thr Leu Ser Val
   485 490
<210> 8
   <211> 491
   <212> PRT
   <213> Marasmius scorodonius
<400> 8
   Phe Pro Asn Phe Ser Lys Ser Ser Ile Leu Lys Pro Arg Arg Thr Asn
   1               5                   10                  15
   Ser Leu Leu Ile Asn Pro Asp Ala Gln Pro Asp Leu Pro Thr Ala Lys
               20                  25                  30
   Gln Ala Ser Thr Ala Ala Ala Ser Val Gly Leu Asn Leu Thr Asp Ile
           35                  40                  45
   Gln Gly Asp Ile Leu Ile Gly Met Lys Lys Asn Lys Glu Met Phe Phe
       50                  55                  60
   Phe Phe Ser Ile Ala Asp Ala Ala Ala Phe Lys Ser His Leu Gly Ser
   65                  70                  75                  80
   Ala Ile Leu Pro Leu Ile Thr Ser Thr Gln Gln Leu Leu Ala Val Ala
                   85                  90                  95
   Ser Gln Pro Thr Thr Ala Val Asn Leu Ala Phe Ser Gln Thr Gly Leu
               100                 105                 110
   Asn Ala Leu Gly Leu Ala Ala Gln Gly Leu Gly Asp Ser Leu Phe Ala
           115                 120                 125
   Ser Gly Gln Phe Ser Gly Ala Gln Ser Leu Gly Asp Pro Gly Thr Ser
       130                 135                 140
   Asn Trp Val Gln Ala Phe Ala Gly Thr Gly Ile His Gly Val Phe Leu
   145                 150                 155                 160
   Leu Ala Ser Asp Thr Val Asp Asn Val Asn Ala Glu Leu Ser Gln Ile
                   165                 170                 175
   Gln Ser Ile Leu Gly Thr Ser Ile Thr Glu Ala Tyr Arg Leu Gln Gly
               180                 185                 190
   Glu Ala Arg Pro Gly Asp Gln Gln Gly His Glu His Phe Gly Phe Met
           195                 200                 205
   Asp Gly Ile Ser Asn Pro Ala Ile Asp Gly Phe Ser Thr Ala Leu Pro
       210                 215                 220
   Gly Gln Ala Val Leu Ser Pro Gly Leu Phe Leu Leu Gly Glu Asp Gly
   225                 230                 235                 240
   Asp Gly Ser Ser Ser Ser Arg Pro Ser Trp Ala Lys Asp Gly Ser Phe
                   245                 250                 255
   Leu Ala Phe Arg Gln Leu Gln Gln Arg Val Pro Glu Phe Asn Lys Phe
               260                 265                 270
   Leu Ala Asp Asn Ala Ala Leu Thr Gln Gly Asn Ala Asp Leu Leu Gly
           275                 280                 285
   Ala Arg Met Met Gly Arg Trp Lys Ser Gly Ala Pro Val Asp Leu Ala
       290                 295                 300
   Pro Thr Ala Asp Asp Val Asp Leu Ala Asn Asp Pro Gln Arg Asn Asn
   305                 310                 315                 320
   Asn Phe Asn Phe Thr His Ala Gly Phe Thr Glu Thr Thr Asp Glu Thr
                   325                 330                 335
   His Cys Pro Phe Ser Ala His Ile Arg Lys Thr Asn Pro Arg Ser Asp
               340                 345                 350
   Phe Asn Pro Gln Asn Thr Asn Asn His Ile Ile Arg Ala Gly Ile Pro
           355                 360                 365
   Tyr Gly Pro Glu Val Thr Asp Ala Glu Ala Ser Ser Asn Thr Ser Ser
       370                 375                 380
   Thr Asp Ala Ser Leu Glu Arg Gly Leu Ala Phe Val Ala Tyr Gln Ser
   385                 390                 395                 400
   Asn Ile Gly Asn Gly Phe Ala Phe Leu Gln Gln Ala Trp Val Asp Asn
                   405                 410                 415
   Ala Asn Phe Phe Phe Gly Lys Thr Thr Pro Pro Gly Val Asp Pro Ile
               420                 425                 430
   Ile Gly Ser Val Ala Ala Gln Asn Asn Phe Ala Pro Asn Gly Pro Arg
           435                 440                 445
   Pro Val Ser Gly Leu Asp Pro Thr Asp Ser Thr Lys Ile Val Thr Ile
       450                 455                 460
   Asn Thr Asp Phe Val Ser Ser Arg Gly Gly Glu Tyr Phe Phe Ser Pro
   465                 470                 475                 480
   Ser Leu Ser Ala Ile Gln Asn Thr Leu Ser Val
                   485                 490